# DEMANDE DE BREVET EUROPEEN

(11) **EP 1 502 576 A2**
(43) Date de publication de la demande: **02.02.2005**
(21) Numéro de dépôt: 04291767.4
(22) Date de dépôt: 12.07.2004
(51) Int. Cl.: A61K 7/06

(54) **Composition contentant un 2-thioacétamide, son utilisation pour stimuler la pousse des fibres kératiniques et/ou freiner leur chute**

(30) Priorité: 31.07.2003 FR 0309476
(71) Demandeur: L'OREAL, 75008 Paris (FR)
(72) Inventeur: Rozot, Roger, 77400 Lagny/ Marne (FR); Boulle, Christophe, Lagny s/ Marne 77400 (FR); Breton, Phillipe, 78150 Le Chesnay (FR)
(74) Mandataire: Wattremez, Catherine

(57) **Abrégé**

L'invention se rapporte à une composition de soin ou de maquillage des cheveux ou des cils, destinée notamment à améliorer leur aspect et/ou augmenter leur densité, contenant une quantité efficace d'un composé 2-thioacétamide de formule (1) ou d'un de ses sels : 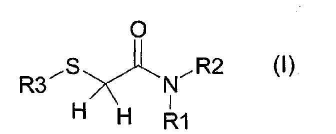 dans laquelle :
a) **R**_{**1**} **et R**_{**2**} représentent indépendamment :
   un atome d'hydrogène ; un radical alkyle en C₁-C₂₀ éventuellement substitué par au moins un substituant ; un cycle hydrocarboné C₁, éventuellement accolé à un cycle C₂ contenant éventuellement au moins un hétéroatome, ces et cycles C₁ et C₂ pouvant comporter au moins une fonction carbonyle ou thiocarbonyle et être substitués ; un hétérocycle Hy₂ éventuellement accolé à un cycle C₂ contenant éventuellement au moins un hétéroatome pour former un hétérocycle Hy₃, ces cycles Hy₂ et C₂ pouvant être substitués et/ou comporter au moins une fonction carbonyle ou thiocarbonyle ; ou un groupe C(=NR)R', C(=NR)NR'R", COR, CSR, COOR, CONRR', SO₂R ou SO₂NRR' ;
**b) R**_{**3**} représente :
   un radical alkyle en C₁-C₂₀ éventuellement substitué par au moins un substituant ; un cycle hydrocarboné C₃, éventuellement accolé à un cycle C₄ contenant éventuellement au moins un hétéroatome, ces cycles C₃ et C₄ pouvant être substitués et/ou comporter au moins une fonction carbonyle ou thiocarbonyle ; un hétérocycle Hy₄ représentant un cycle pyrrole, furanne, thiophène ou pyrazole, éventuellement accolé à un cycle C₅ représentant un cycle phényle, pyridine ou pyrimidine, ces cycles Hy₄ et C₅ pouvant être substitués ; un cycle pyridine éventuellement accolé à un cycle C₂ contenant éventuellement au moins un hétéroatome, ces cycles pyridine et C₂ pouvant être substitués et/ou comporter au moins une fonction carbonyle ou thiocarbonyle ; un hétérocycle Hy₅ différent de Hy₄ et du cycle pyridine, éventuellement accolé à un cycle C₂ contenant éventuellement au moins un hétéroatome, ces cycles Hy₅ et C₂ pouvant être substitués et/ou comporter au moins une fonction carbonyle ou thiocarbonyle ; ou un groupe C(=NR)R', C(=NR)NR'R", COR, CSR, COOR ou CONRR'.

## Description

L'invention a pour objet une composition de soin ou de maquillage des fibres kératiniques humaines, contenant un composé 2-thioacétamide, destinée à induire et/ou stimuler la croissance des fibres kératiniques et/ou freiner leur chute. Elle se rapporte, en outre, à un procédé de traitement cosmétique destiné à stimuler la croissance des fibres kératiniques et/ou freiner leur chute.

Plus spécialement, l'invention a trait à une composition de soin ou de maquillage des cheveux ou des cils, contenant une quantité efficace d'un 2-thioacétamide, destinée à augmenter leur densité et/ou améliorer leur aspect.

La croissance des cheveux et leur renouvellement sont principalement déterminés par l'activité des follicules pileux et de leur environnement matriciel. Leur activité est cyclique et comporte essentiellement trois phases à savoir la phase anagène, la phase catagène et la phase télogène.

A la phase anagène (phase active ou de croissance), qui dure plusieurs années et au cours de laquelle les cheveux s'allongent, succède une phase catagène très courte et transitoire qui dure quelques semaines. Au cours de cette phase, le cheveu subit une évolution, le follicule s'atrophie et son implantation dermique apparaît de plus en plus haute.

La phase terminale ou phase télogène, qui dure quelques mois, correspond à une phase de repos du follicule et le cheveu finit par tomber. A la fin de cette période de repos, un nouveau follicule est régénéré, sur place, et un autre cycle recommence.

La chevelure se renouvelle donc en permanence et sur les 150 000 cheveux environ que comporte une chevelure, 10% environ sont au repos et seront remplacés en quelques mois.

La chute ou perte naturelle des cheveux peut être estimée, en moyenne, à quelques cent cheveux par jour pour un état physiologique normal. Ce processus de renouvellement physique permanent subit une évolution naturelle au cours du vieillissement, les cheveux deviennent plus fins et leurs cycles plus courts.

En outre, différentes causes peuvent entraîner une perte importante, temporaire ou définitive, des cheveux. Il peut s'agir de chute et d'altération des cheveux au décours d'une grossesse (post partum), au cours d'états de dénutrition ou de déséquilibres alimentaires ou encore au cours d'états d'asthénie ou de dysfonctionnement hormonal comme cela peut être le cas au cours ou au décours de la ménopause. Il peut également s'agir de chute ou d'altérations des cheveux en relation avec des phénomènes saisonniers.

Il peut s'agir également d'une alopécie, qui est essentiellement due à une perturbation du renouvellement capillaire entraînant dans un premier temps l'accélération de la fréquence des cycles au détriment de la qualité des cheveux, puis de leur quantité. Les cycles de croissance successifs aboutissent à des cheveux de plus en plus fins et de plus en plus courts, se transformant peu à peu en un duvet non pigmenté, entraînant ainsi un appauvrissement progressif de la chevelure. Des zones sont touchées préférentiellement, notamment les golfes temporaux ou frontaux chez l'homme et, chez la femme, on constate une alopécie diffuse du vertex.

Le terme alopécie recouvre aussi toute une famille d'atteintes du follicule pileux ayant pour conséquence finale la perte définitive, partielle ou générale des cheveux. Il s'agit plus particulièrement de l'alopécie androgénique. Dans un nombre important de cas, la chute précoce des cheveux survient chez des sujets prédisposés génétiquement, il s'agit alors d'alopécie andro-chrono-génétique ; cette forme d'alopécie concerne notamment les hommes.

Il est connu, par ailleurs, que certains facteurs tels qu'un déséquilibre hormonal, un stress physiologique, la malnutrition, peuvent accentuer le phénomène.

Dans certaines dermatoses du cuir chevelu à caractéristique inflammatoire, telles que par exemple le psoriasis ou les dermatites séborrhéiques, la chute des cheveux peut être fortement accentuée ou entraîner des cycles des follicules fortement perturbés.

On recherche depuis de nombreuses années, dans l'industrie cosmétique ou pharmaceutique, des compositions permettant de supprimer ou de réduire l'alopécie, et notamment d'induire ou de stimuler la croissance des cheveux ou de diminuer leur chute.

Dans cette optique, on a déjà proposé un grand nombre de compositions comprenant des actifs très divers, comme par exemple le 2,4-diamino 6-pipéridinopyrimidine 3-oxyde ou "minoxidil" décrit dans les brevets US 4 139 619 et US 4 596 812 ou encore ses nombreux dérivés comme ceux décrits par exemple dans les demandes de brevet EP 0353123, EP 0356271, EP 0408442, EP 0522964, EP 0420707, EP 0459890, EP 0519819.

Des études cliniques ont démontré que des analogues de PGF2-α avaient la propriété de provoquer la croissance de poils et de cils chez l'homme et chez l'animal (Murray A. and Johnstone M.D., 1997. *Am. J. Opht*., 124(4), 544-547). Chez l'homme, des essais réalisés sur le cuir chevelu ont montré qu'un analogue de prostaglandine E2 (le viprostol) avait la propriété d'augmenter la densité capillaire (Roenigk H.H., 1988. *Clinic Dermatol*., 6(4), 119-121).

Par ailleurs, le brevet WO 98/33497 décrit des compositions pharmaceutiques contenant des prostaglandines ou des dérivés de prostaglandines, destinées à lutter contre la chute des cheveux chez l'homme. Les prostaglandines du type A2, F2α et E2 sont mentionnées comme préférées.

Cependant, les prostaglandines sont des molécules au temps de demi-vie biologique très court et agissant de façon autocrine ou paracrine, ceci traduisant le caractère local et labile du métabolisme des prostaglandines (Narumiya S. et *al*., 1999, *Physiol. Rev.,* 79(4), 1193-1226).

Il apparaît donc comme important, pour maintenir et/ou augmenter la densité capillaire chez l'homme de préserver les réserves endogènes de PGF2-α comme de PGE2 des différents compartiments du follicule pileux ou de son environnement cutané proche.

Une solution donnant de bons résultats est l'emploi de composés inhibiteurs de lipoxygénase et/ou inducteurs de la cyclo-oxygénase en vue de favoriser la croissance des cheveux ; une hypothèse est que l'emploi de tels composés oriente le métabolisme des acides gras vers la synthèse endogène de prostaglandines de préférence à d'autres voies.

Toutefois, pour améliorer encore les résultats, il serait souhaitable de pouvoir prolonger l'activité des prostaglandines impliquées dans la croissance et le maintien du cheveu en vie.

Il est par ailleurs bien connu que les programmes de différenciation des kératinocytes de l'épiderme et du follicule pileux sont clairement différents. Ainsi, il est connu que les kératines de la tige pilaire représente une famille (Langbein et al., 2001, J. Biol. Chem. 276 : 35123-35132) distincte de celle exprimée dans l'épiderme, que les marqueurs de différenciation tels que les kératines K₁ et K₁₀ ne sont pas exprimés dans le follicule pileux et en particulier dans la gaine externe (Lenoir et al., 1988, Dev. Biol. 130 : 610-620), que la trichohyaline (O'Guin et al., 1992, J. Invest. Dermatol. 98 : 24-32) et la kératine K6irs (Porter et al., 2001, Br. J. Dermatol. 145 : 558-568) sont exprimées dans le follicule pileux en particulier dans la gaine interne mais pas dans l'épiderme, et que la cyclo-oxygénase de type 1, si elle est exprimée dans l'épiderme, ne l'est pas dans les kératinocytes du follicule pileux mais dans la papille dermique (Michelet. et al., 1997, J. Invest. Dermatol. 108 : 205-209).

Le demandeur a maintenant mis en évidence qu'une enzyme spécifiquement impliquée dans la dégradation de ces prostaglandines est présente dans la papille dermique du cheveu, qui est un compartiment déterminant pour la vie du cheveu. En effet, le demandeur a maintenant prouvé la présence de 15-hydroxy prostaglandine déshydrogénase (15-PGDH en abréviation) à ce niveau. Il a en outre montré que l'inhibition de la 15-PGDH a un effet bénéfique sur la croissance pilaire.

C'est pourquoi la présente invention se rapporte à une composition de soin ou de traitement des fibres kératiniques humaines et notamment des cheveux humains contenant au moins un inhibiteur particulier de la 15-hydroxy prostaglandine déshydrogénase et un milieu physiologiquement acceptable.

La 15-PGDH est une enzyme clé dans la désactivation des prostaglandines, en particulier de la PGF2-α, et de la PGE2, qui sont des médiateurs importants de la croissance et la survie du cheveu. Elle répond à la classification EC 1.1.1.141 et est NAD+ dépendante. Elle a été isolée de rein de porc ; on a notamment observé son inhibition par une hormone thyroïdienne, la tri-iodo thyronine, à des doses très supérieures aux doses physiologiques.

Cependant, il n'avait jamais été proposé d'utiliser un inhibiteur de 15-PGDH pour maintenir et/ou augmenter la densité des fibres kératiniques humaines et notamment les cheveux humains et/ou pour réduire l'hétérogénéité des diamètres des fibres kératiniques humaines et notamment des cheveux humains. Par augmenter la densité des fibres kératiniques, et notamment la densité capillaire, on entend augmenter le nombre de fibres kératiniques et notamment de cheveux par cm² de peau ou cuir chevelu.

Le demandeur a trouvé que certains composés 2-thioacétamides, salifiés ou non sont d'une façon surprenante dotés d'une activité favorable à l'amélioration de la densité des fibres kératiniques humaines. Il a par ailleurs trouvé que ces composés sont des inhibiteurs de la 15-hydroxy prostaglandine déshydrogénase.

La présente invention a donc pour objet une composition à application topique, contenant dans un milieu physiologiquement acceptable une quantité efficace d'un composé 2-thioacétamide de formule (I) ou d'un de ses sels : 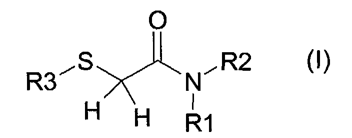 dans laquelle :
**a) R**_{**1**} **et R**_{**2**} représentent indépendamment :
   1) un atome d'hydrogène,
   2) un radical alkyle en C₁-C₂₀ éventuellement substitué par au moins un substituant A₁,
   3) un cycle hydrocarboné C₁, éventuellement accolé à un cycle C₂ contenant éventuellement au moins un hétéroatome pour former un hétérocycle Hy₁, ces cycles C₁ et C₂ pouvant comporter au moins une fonction carbonyle ou thiocarbonyle et être substitués par au moins un substituant A₃,
   4) un hétérocycle Hy₂ éventuellement accolé à un cycle C₂ contenant éventuellement au moins un hétéroatome pour former un hétérocycle Hy₃, ces cycles Hy₂ et C₂ pouvant comporter une fonction carbonyle ou thiocarbonyle et être substitués par au moins un substituant A₃, ou
   5) un groupe C(=NR)R', C(=NR)NR'R", COR, CSR, COOR, CONRR', SO₂R ou SO₂NRR' ;
**b) R**_{**3**} représente :
   1) un radical alkyle en C₁-C₂₀ éventuellement substitué par au moins un substituant A₂,
   2) un cycle hydrocarboné C₃, éventuellement accolé à un cycle C₄ contenant éventuellement au moins un hétéroatome pour former un hétérocycle Hy₃, ces cycles C₃ et C₄ pouvant comporter au moins une fonction carbonyle ou thiocarbonyle et être substitués par au moins un substituant A₃,
   3) un hétérocycle Hy₄ représentant un cycle pyrrole, furanne, thiophène ou pyrazole, éventuellement accolé à un cycle C₅ représentant un cycle phényle, pyridine ou pyrimidine, ces deux cycles Hy₄ et C₅ pouvant être substitués par au moins un substituant A₃,
   4) un cycle pyridine éventuellement accolé à un cycle C₂ contenant éventuellement au moins un hétéroatome pour former un hétérocycle Hy₁, ces cycles pyridine et C₂ pouvant comporter au moins une fonction carbonyle ou thiocarbonyle et être substitués par au moins un substituant A₄,
   5) un hétérocycle Hy₅ différent de Hy₄ et du cycle pyridine, éventuellement accolé à un cycle C₂ contenant éventuellement au moins un hétéroatome pour former un hétérocycle Hy₁, ces cycles Hy₅ et C₂ pouvant comporter au moins une fonction carbonyle ou thiocarbonyle et être substitués par au moins un substituant A₃, ou
   6) un groupe C(=NR)R', C(=NR)NR'R", COR, CSR, COOR ou CONRR' ;
**c) A**_{**1**} représente :
   1) un halogène,
   2) un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R",
   3) un cycle hydrocarboné C₆, éventuellement accolé à un cycle C₇ contenant éventuellement au moins un hétéroatome pour former un hétérocycle Hy₆, ces cycles C₆ et C₇ pouvant comporter au moins une fonction carbonyle ou thiocarbonyle et être substitués par au moins un substituant A₅,
   4) un hétérocycle Hy₇ éventuellement accolé à un cycle C₇ contenant éventuellement au moins un hétéroatome pour former un hétérocycle Hy₆, ces cycles Hy₇ et C₇ pouvant comporter au moins une fonction carbonyle ou thiocarbonyle et être substitués par au moins un substituant A₅ ;
**d) A**_{**2**} représente :
   1) un halogène,
   2) un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R", ou
   3) un cycle C₈ éventuellement accolé à un cycle C₉, ces cycles C₈ et C₉ contenant éventuellement au moins un hétéroatome pour former un hétérocycle Hy₈ et/ou au moins une fonction carbonyle ou thiocarbonyle et/ou étant substitués par au moins un substituant A₅ ;
**e) A**_{**3**} représente :
   1) A₂ , ou
   2) un groupe alkyle en C₁-C₂₀ éventuellement substitué par au moins un substituant A₅ ;
**f) A**_{**4**} représente :
   1) un halogène,
   2) un groupe CF₃, CN, OR, SR, NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R",
   3) un groupe alkyle en C₁-C₂₀ éventuellement substitué par au moins un substituant A₅,
   4) un cycle C₈ éventuellement accolé à un cycle C₉, ces cycles C₈ et C₉ contenant éventuellement au moins un hétéroatome pour former un hétérocycle Hy₈ et/ou au moins une fonction carbonyle ou thiocarbonyle et/ou étant substitués par au moins un substituant A₅ ;
**g) A**_{**5**} représente :
   1) un halogène,
   2) un groupe CF₃, CN, OR₅, SR₅, NR₅R₅', NR₅C(=NR₅')NR₅"R₅"', COR₅, CSR₅, COOR₅, CONR₅R₅', CSNR₅R₅', NR₅CSR₅', NR₅CSNR₅'R₅", NR₅COR₅', NR₅CONR₅'R₅", SO₂NR₅R₅', NR₅SO₂R₅', SO₂R₅ ou SiR₅R₅'R₅", R₅, R₅', R₅" étant un atome d'hydrogène ou un groupe alkyle en C₁-C₂₀,
   3) un groupe alkyle en C₁-C₂₀, ou
   4) un cycle C₁₀ éventuellement accolé à un autre cycle C₁₁, ces cycles contenant éventuellement au moins un hétéroatome pour former un hétérocycle Hy₉ et/ou comportant au moins une fonction carbonyle ou thiocarbonyle ;
h) R, R', R" et R"', identiques ou différents, représentent :
   1) un atome d'hydrogène,
   2) un groupe alkyle en C₁-C₂₀ éventuellement substitué par au moins un substituant A₅, ou
   3) un cycle C₁₂ éventuellement accolé à un autre cycle C₁₃, ces cycles contenant éventuellement au moins un hétéroatome pour former un hétérocycle Hy₁₀ et pouvant être substitués par au moins un substituant A₅ ;
**i) Hy**_{**1**} **à Hy**_{**10**} représentent indépendamment un hétérocycle pouvant contenir de 1 à 4 hétéroatomes choisis parmi N, O et S.

L'invention se rapporte encore à l'utilisation notamment cosmétique d'au moins un composé 2-thioacétamide de formule (I) ou d'un de ses sels, tel que défini précédemment, comme agent pour induire et/ou stimuler la croissance des fibres kératiniques humaines notamment les cils et les cheveux et/ou freiner leur chute et/ou augmenter leur densité.

Les fibres kératiniques humaines auxquelles s'applique l'invention sont notamment les cheveux, les sourcils, les cils, les poils de barbe, de moustache et les poils pubiens. Plus spécialement, l'invention s'applique aux cheveux et/ou aux cils humains.

Aussi, l'invention se rapporte encore à l'utilisation cosmétique d'au moins un composé 2-thioacétamide de formule (I) ou de l'un de ses sels, dans une composition cosmétique de soin capillaire d'être humain pour réduire la chute des cheveux et/ou augmenter leur densité et/ou traiter l'alopécie andro-chrono-génétique. Elle a encore pour objet l'utilisation d'au moins un composé 2-thioacétamide de formule (I) ou d'un de ses sels pour la préparation d'une composition capillaire pour être humain, destinée à induire et/ou stimuler la croissance des cheveux et/ou freiner leur chute et/ou augmenter leur densité et/ou traiter l'alopécie androgénique.

L'invention se rapporte encore à l'utilisation cosmétique d'au moins un composé 2-thioacétamide de formule (I) ou d'un de ses sels, dans une composition cosmétique de soin capillaire d'être humain pour traiter l'alopécie d'origine naturelle et en particulier androgénique ainsi qu'à l'utilisation d'au moins un composé 2-thioacétamide de formule (I) ou d'un de ses sels pour la préparation d'une composition de soin capillaire d'être humain, destinée à traiter l'alopécie d'origine naturelle et en particulier androgénique. Ainsi, cette composition permet de maintenir en bon état la chevelure et/ou lutter contre la chute naturelle des cheveux et plus spécialement celle des hommes.

L'invention a encore pour objet l'utilisation cosmétique d'au moins un composé 2-thioacétamide de formule (I) ou d'un de ses sels, dans une composition cosmétique de soin et/ou de maquillage des cils d'être humain, pour induire et/ou stimuler la croissance des cils et/ou augmenter leur densité ainsi que l'utilisation d'au moins un composé 2-thioacétamide de formule (I) ou d'un de ses sels, pour la préparation d'une composition de soin et/ou de traitement des cils d'être humain, destinée à induire et/ou stimuler la croissance des cils et/ou augmenter leur densité. Cette composition permet ainsi de maintenir en bon état les cils et/ou améliorer leur état et/ou leur aspect.

L'invention a encore pour objet l'utilisation d'au moins un composé 2-thioacétamide de formule (I) ou d'un de ses sels comme inhibiteur de la 15-hydroxy prostaglandine déshydrogénase. Elle a encore pour objet l'utilisation d'au moins un composé 2-thioacétamide de formule (I) ou d'un de ses sels pour la fabrication d'une composition destinée à traiter les désordres liés à la présence de 15-hydroxy prostaglandine déshydrogénase chez l'être humain.

Par inhibiteur de la 15-hydroxy prostaglandine déshydrogénase, on entend un composé de formule (I), capable d'inhiber ou de diminuer l'activité de l'enzyme 15-PGDH, et/ou capable d'inhiber, diminuer ou ralentir la réaction catalysée par cette enzyme.

Selon un mode de réalisation avantageux de l'invention, le composé de formule (I) est un inhibiteur spécifique de la 15-PGDH ; par inhibiteur spécifique on entend un actif qui est peu ou pas inhibiteur de la synthèse des prostaglandines, en particulier de la synthèse de PGF2-α ou de PGE2. Selon un mode particulier de mise en oeuvre de l'invention, l'inhibiteur de la 15-PGDH est peu ou n'est pas inhibiteur de la prostaglandine synthase (PGF synthase).

En effet, le demandeur a trouvé que la PGF synthase est également exprimée dans la papille dermique. Le maintien d'une quantité efficace de prostaglandines au site d'action résulte donc d'un équilibre biologique complexe entre la synthèse et la dégradation de ces prostaglandines. L'apport exogène de composés inhibant le catabolisme sera donc moins efficace si cette activité est combinée à une inhibition de la synthèse de ces prostaglandines.

Avantageusement, les composés de formule (I), sous forme salifiée ou non, présentent une activité inhibitrice de la 15-PGDH supérieure à l'activité d'inhibition de la PGF synthase. En particulier, le rapport entre les activités inhibitrices respectivement de la PGF synthase et de la 15-PGDH pour une concentration donnée, déterminées notamment par les concentrations inhibitrices de 50% de l'activité enzymatique, respectivement de la PGF synthase (IC_{50fs}) et de la 15-PGDH (IC_{50dh}) est au moins supérieur à 1, et notamment d'au moins 3:1, avantageusement supérieur ou égal à 5:1. Les composés préférés de l'invention présentent un ratio IC_{50fs}/IC_{50dh} supérieur ou égal à 10:1, et en particulier supérieur ou égal à 15.

Dans la suite du texte, et sauf mention exprès, l'emploi du terme composé de formule (I) doit être compris comme signifiant aussi bien le composé de formule (I) sous forme acide ou basique, que l'un de ses sels. Il peut aussi être sous forme tautomère.

"Au moins un" selon l'invention signifie un ou plusieurs (2, 3 ou plus). En particulier, la composition peut contenir un ou plusieurs composés de formule (I). Ce ou ces composés peuvent être des isomères cis ou trans ou un mélange d'isomères cis/trans. Ils peuvent aussi être sous forme tautomère. Ce ou ces composés peuvent être des énantiomères et/ou des diastéréoisomères ou un mélange de ces isomères, en particulier un mélange racémique.

Par "hydrocarboné", on entend au sens de l'invention un groupement d'atomes d'hydrogène et de carbone.

Par "radical alkyle" on entend au sens de l'invention un radical hydrocarboné qui peut être linéaire ou ramifié et saturé ou insaturé. En particulier, le radical alkyle comporte de 1 à 20 atomes de carbone, de préférence de 1 à 10. Comme exemple de radical alkyle utilisable dans l'invention, on peut citer les radicaux méthyle, éthyle, isopropyle, n-butyle, *ter*-butyle, n-hexyle, éthyle-2-hexyle, éthylène, propylène.

Selon l'invention, les cycles C₁ à C₁₃, Hy₂, Hy₄ et Hy₅ de la formule (I) comportent de 3 à 7 atomes et mieux de 5 à 6 atomes et peuvent être saturés ou insaturés. En outre, les cycles C₂, C₄, C₇, C₈, C₉, C₁₀, C₁₁, C₁₂ et C₁₃ peuvent comporter éventuellement un ou plusieurs hétéroatomes tels que S, N, O ou leurs associations et former ainsi des hétérocycles. Hy₇ représente un hétérocycle pouvant comporter de 1 à 4 hétéroatomes choisis parmi N, S, O et leur association et comportant de 3 à 15 atomes et mieux de 5 à 6 atomes. Selon un mode particulier de l'invention, l'hétérocycle Hy₇ peut comporter jusqu'à 15 atomes comme un éther-couronne possédant 5 motifs -CH₂CH₂O-. Ces cycles peuvent en outre être accolés à un autre cycle de nature chimique identique ou différente. En outre, ces cycles peuvent être substitués en particulier par un substituant A₃ ou A₅.

Comme cycles hydrocarbonés saturés utilisables dans l'invention on peut citer le radical cyclopropyle, cyclobutyle, cyclopentyle, cyclohexyle ou cycloheptyle. Comme cycles hydrocarbonés insaturés, on peut citer le cycle cyclohexényle ou phényle. Comme cycles hydrocarbonés accolés, utilisables, on peut citer le radical naphtyle.

Selon l'invention, R₁ et/ou R₂ peuvent représenter un cycle hydrocarboné tel que défini ci-dessus, en particulier saturé. Selon l'invention R₁ et/ou R₂ peuvent aussi représenter un hétérocycle Hy₂ comportant de 3 à 7 atomes et mieux de 5 à 6 atomes, et comportant de 1 à 4 hétéroatomes choisi parmi N, O, S et leur association. Cet hétérocycle peut, en outre comporter, une ou plusieurs fonctions carbonyle ou thiocarbonyle. A titre d'exemple Hy₂ représente l'un des hétérocycles suivants : azétidine, pyrrole, dihydropyrrole, pyrrolidine, furanne, dihydrofuranne, tétrahydrofuranne, thiophène, dihydrothiophène, tétrahydrothiophène, imidazole, dihydro-imidazole, imidazolidine, dihydrothiazole, thiazolidine, dihydropyrazole, pyrazolidine, oxazole, dihydro-oxazole, oxazolidine, isoxazole, dihydro-isoxazole, isoxazolidine, isothiazole, dihydro-isothiazole, isothiazolidine, triazole, dihydrotriazole, triazolidine, oxadiazole, dihydro-oxadiazole, oxadiazolidine, thiadiazole, dihydrothiadiazole, thiadiazolidine, tétrazole, pyridine, dihydropyridine, tétrahydropyridine, pipéridine, pyranne, dihydropyranne, tétrahydropyranne, pyrimidine, dihydropyrimidine, tétrahydropyrimidine, pipérazine, pyridazine, pyrazine, triazine, morpholine, azépine, diazépine. De préférence, Hy₂ représente un cycle pypéridine. Cet hétérocycle Hy₂ peut, en outre, être accolé à un cycle C₂ de 4 à 7 atomes, saturé ou insaturé, C₂ contenant éventuellement au moins un hétéroatome choisi parmi O, N, S pour former un hétérocycle Hy₁. C₂ peut en outre comporter une ou plusieurs fonctions carbonyle ou thiocarbonyle. Comme exemple d'hétérocycle Hy₁ utilisable dans l'invention, on peut citer les cycles pyridine, pipéridine, morpholine, pyrrole, furanne, thiophène, imidazole, oxazole, thiazole, pyrazole, pyrimidine, pipérazine, pyrazine, pyridazine, triazine, pyrrolidine, thiazolidine.

Selon l'invention, R₁ et/ou R₂ peuvent, en outre, représenter un radical alkyle dont l'un au moins des hydrogènes est substitué par un hétérocycle Hy₇. Cet hétérocycle peut comporter de 3 à 7 atomes, et mieux de 5 à 6 atomes, et comprendre de 1 à 4 hétéroatomes choisis parmi N, O, S et leur association. Selon un mode particulier de l'invention, cet hétérocycle Hy₇ peut comporter jusqu'à 15 atomes comme un éther couronne à 5 motifs -CH₂CH₂O-. Selon un autre mode, Hy₇ comporte une fonction carbonyle. Comme hétérocycle Hy₇ utilisable dans l'invention, on peut citer les cycles azétidine, pyrrole, dihydropyrrole, pyrrolidine, furanne, dihydrofuranne, tétrahydrofuranne, thiophène, dihydrothiophène, tétrahydrothiophène, imidazole, dihydro-imidazole, imidazolidine, thiazole, dihydrothiazole, thiazolidine, pyrazole, dihydropyrazole, pyrazolidine, oxazole, dihydro-oxazole, oxazolidine, isoxazole, dihydro-isoxazole, isoxazolidine, isothiazole, dihydro-isothiazole, isothiazolidine, triazole, dihydrotriazole, triazolidine, oxadiazole, dihydro-oxadiazole, oxadiazolidine, thiadiazole, dihydrothiadiazole, thiadiazolidine, tétrazole, pyridine, dihydropyridine, tétrahydropyridine, pyranne, dihydropyranne, tétrahydropyranne, pyrimidine, dihydropyrimidine, tétrahydropyrimidine, pipérazine, pyridazine, pyrazine, triazine, morpholine, azépine, diazépine, éther couronne à 15 atomes et 5 motifs -CH₂CH₂O-, pyrrolidinone. De préférence, Hy₇ représente un des cycles pyrrole, oxyazole, pyridine, furanne, pyrrolidine, morpholine, tétrahydrofuranne, éther couronne à 15 atomes (5 motifs -CH₂CH₂O) ou pyrrolidinone.

Selon l'invention R₃ peut représenter un cycle hydrocarboné C₃ de 3 à 7 atomes de carbone, saturé ou insaturé, et mieux de 5 à 6 atomes de carbones, éventuellement accolé à un hétérocycle Hy₃. Cet hétérocycle Hy₃ peut comporter de 4 à 7 atomes et mieux de 5 à 6 atomes et comprendre de 1 à 4 hétéroatomes choisis parmi N, O, S et leur combinaison. En outre, cet hétérocycle Hy₃ peut comporter une ou plusieurs fonctions carbonyle ou thiocarbonyle.

Comme hétérocycle Hy₃ utilisable dans l'invention on peut citer les cycles azétidine, pyrrole, dihydropyrrole, pyrrolidine, furanne, dihydrofuranne, tétrahydrofuranne, thiophène, dihydrothiophène, tétrahydrothiophène, imidazole, dihydro-imidazole, imidazolidine, dihydrothiazole, thiazolidine, pyrazole, dihydropyrazole, pyrazolidine, oxazole, dihydro-oxazole, oxazolidine, isoxazole, dihydro-isoxazole, isoxazolidine, isothiazole, dihydro-isothiazole, isothiazolidine, triazole, dihydrotriazole, triazolidine, oxadiazole, dihydro-oxadiazole, oxadiazolidine, thiadiazole, dihydrothiadiazole, thiadiazolidine, tétrazole, pyridine, dihydropyridine, tétrahydropyridine, pipéridine, pyranne, dihydropyranne, tétrahydropyranne, pyrimidine, dihydropyrimidine, tétrahydropyrimidine, pipérazine, pyridazine, pyrazine, triazine, morpholine, azépine, diazépine. De préférence, Hy₃ représente un des cycles pyrrole, pyridine, pyrimidine, imidazole, triazole, furanne, thiophène, oxazole, thiazole.

Selon l'invention, R₃ peut aussi représenter :
- un cycle pyridine éventuellement accolé à un cycle C₂ de nature chimique identique ou différente,
- un hétérocycle Hy₄ choisi parmi les cycles pyrrole, furanne, thiophène et pyrazole éventuellement accolé à un cycle phényle, pyridine ou pyrimidine,
- un autre hétérocycle Hy₅ comportant de 3 à 7 atomes, et mieux de 5 à 6 atomes, et de 1 à 4 hétéroatomes choisi parmi N, O, S et leur association. Cet hétérocycle Hy₅ peut, en outre, comporter une ou plusieurs fonctions carbonyle ou thiocarbonyle et être accolé à un autre cycle C₂ de nature chimique identique ou différente.

A titre d'exemple, l'hétérocycle Hy₅ est choisi parmi les cycles azétidine, dihydropyrrole, pyrrolidine, dihydrofuranne, tétrahydrofuranne, dihydrothiophène, tétrahydrothiophène, imidazole, dihydro-imidazole, imidazolidine, thiazole, dihydrothiazole, thiazolidine, dihydropyrazole, pyrazolidine, oxazole, dihydro-oxazole, oxazolidine, isoxazole, dihydro-isoxazole, isoxazolidine, isothiazole, dihydro-isothiazole, isothiazolidine, triazole, dihydrotriazole, triazolidine, oxadiazole, dihydro-oxadiazole, oxadiazolidine, thiadiazole, dihydrothiadiazole, thiadiazolidine, tétrazole, dihydropyridine, tétrahydropyridine, pipéridine, pyranne, dihydropyranne, tétrahydropyranne, pyrimidine, dihydropyrimidine, tétrahydropyrimidine, pipérazine, pyridazine, pyrazine, triazine, morpholine, azépine, diazépine, 2-benzothiazolyle, thiazolo[2,3-c][1,2,4]triazole. De préférence, Hy₅ représente un des cycles 2-benzothiazolyle, thiazolo[2,3-c][1,2,4]triazole, imidazole, thiazole, triazole, oxazole, pyrimidine, pyrazine, pyridazine.

Comme exemple d'hétérocycle Hy₁, Hy₆, Hy₈, Hy₉, Hy₁₀ utilisable dans l'invention, on peut citer indépendamment les cycles azétidine, pyrrole, dihydropyrrole, pyrrolidine, furanne, dihydrofuranne, tétrahydrofuranne, thiophène, dihydrothiophène, tétrahydrothiophène, imidazole, dihydro-imidazole, imidazolidine, thiazole, dihydrothiazole, thiazolidine, pyrazole, dihydropyrazole, pyrazolidine, oxazole, dihydrooxazole, oxazolidine, isoxazole, dihydro-isoxazole, isoxazolidine, isothiazole, dihydroisothiazole, isothiazolidine, triazole, dihydrotriazole, triazolidine, oxadiazole, dihydrooxadiazole, oxadiazolidine, thiadiazole, dihydrothiadiazole, thiadiazolidine, tétrazole, pyridine, dihydropyridine, tétrahydropyridine, pipéridine, pyranne, dihydropyranne, tétrahydropyranne, pyrimidine, dihydropyrimidine, tétrahydropyrimidine, pipérazine, pyridazine, pyrazine, triazine, morpholine, azépine, diazépine. De préférence, on utilise les cycles pyrrole, pyrrolidine, imidazole, furanne, pyrazole, pyridine, pyrimidine, triazole, pyrazine, pyridazine, pipéridine, pipérazine, morpholine.

Les hétérocycles de la formule (I) peuvent, en outre, être accolés à un cycle de nature chimique identique ou différente.

Comme hétérocycles accolés, utilisables dans l'invention on peut citer les cycles purine, ptéridine. Comme hétérocycles accolés à un cycle hydrocarboné, utilisables dans l'invention dans l'invention on peut citer, les radicaux benzofuranne, benzothiophène, benzothiazole, indole, benzimidazole, quinoline, isoquinoline, quinazoline.

Comme atome d'halogène utilisable dans l'invention, on peut citer les atomes de chlore, de fluor ou de brome, et mieux les atomes de fluor et de chlore.

Selon l'invention, les composés de formule (I) sont sous forme isolée, c'est-à-dire non polymérique.

Selon un mode particulier de réalisation de l'invention, l'un au moins des R₁ et R₂ représente un atome d'hydrogène ; un radical alkyle en C₁-C₂₀ dont au moins un hydrogène est substitué par au moins un substituant A₁ ; un cycle hydrocarboné saturé ou non de 3 à 6 atomes de carbone, dont au moins un hydrogène est éventuellement substitué par le groupe halogène comme CF₃ ou des atomes d'halogènes et notamment par 1 ou 2 atomes de chlore ou de brome lorsque le cycle est un phényle. Ces groupe et atomes d'halogènes peuvent être situés en position para, méta ou ortho par rapport à l'atome d'azote portant R₁ et R₂. Le cycle hydrocarboné est notamment un radical cyclopentyle, cyclopropyle, cyclobutyle, cyclohexyle ou un radical aryle comme le phényle. En particulier R₁ représente H et R₂ représente un radical cyclopentyle, cyclopropyle, cyclobutyle, cyclohexyle ou un radical phényle substitué par un atome de brome ou 2 atomes de chlore.

Selon un autre mode de réalisation, R₁ représente H et R₂ représente un hétérocycle de 5 à 6 atomes, saturé ou insaturé, contenant de 1 à 4 hétéroatomes choisis parmi N, O, S et éventuellement substitué par au moins un radical alkyle, CF₃, OR, SR, NRR', COR, COOR, CONRR' ou NRCOR' avec R et R' représentant H ou alkyle. Par exemple R₂ représente un cycle pipéridine éventuellement substitué par au moins un radical alkyle en C₁-C₁₀, et notamment méthyle.

Selon un autre mode de réalisation, R₁ représente H et R₂ représente un alkyle en C₁-C₂₀, saturé, éventuellement substitué par au moins un substituant A₁. En particulier, A₁ représente un cycle de 5 à 6 atomes, saturé ou insaturé, contenant éventuellement de 1 à 4 hétéroatomes choisis parmi N, O, S, pouvant contenir au moins une fonction carbonyle et être éventuellement substitué par au moins un groupe choisi parmi alkyle, F, CF₃, OR, SR, NRR', COR, COOR, CONRR' ou NRCOR' avec R et R' représentant H ou alkyle ; un cycle éther-couronne à 15 atomes et 5 motifs -CH₂CH₂O- ; un groupe choisi parmi CF₃, OR, SR, NRR', COR, COOR, CONRR', NRCOR' ou SiRR'R" avec R, R' et R" représentant H ou alkyle.

Selon un autre mode de réalisation de l'invention, R₃ représente un phényle ou un hétérocycle de 5 à 6 atomes, substitué par un ou deux substituant A₃ ou accolé à un cycle hydrocarboné ou hétérocyclique de 5 à 6 atomes. En particulier, R₃ représente un hétérocycle comportant comme hétéroatome au moins un atome d'azote et éventuellement un atome de soufre, cet hétérocycle étant éventuellement accolé à un cycle phényle ou thiazole lui-même éventuellement accolé à un cycle hydrocarboné, notamment phényle, ou substitué par un groupe COR ou CN ou deux groupes respectivement COR et CN. En particulier, R₃ représente un groupe phényle, 2-benzothiazolyle, 2-pyridyle, 6-acétyl-nicotinonitrile, triazolyle ou 4a,8a-dihydrobenzo[4,5]thiazolo[2,3-c][1,2,4]triazolyle.

Selon un mode particulier de réalisation de l'invention, le composé 2-thioacétamide présente une des formules (II) et (III) suivantes : 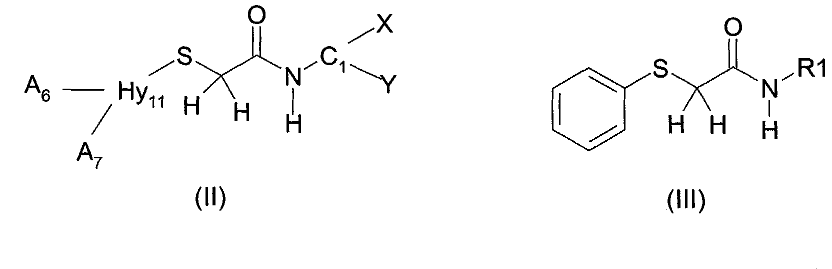 dans lesquelles X et Y représentent indépendamment un atome d'hydrogène ou un halogène ; C₁ représente un cycle hydrocarboné de 3 à 6 à atomes de carbone, saturé ou insaturé ; Hy₁₁ représente un hétérocycle de 5 à 6 atomes contenant au moins un hétéroatome choisi parmi N, S et leur combinaison ; A₆ et A₇ représentent indépendamment un substituant choisi parmi hydrogène, alkyle, COR, OR, SR, CN, COOR et les cycles C₈ à 5 ou 6 atomes, saturés ou non comportant éventuellement de 1 à 4 hétéroatomes choisis parmi S, N et leurs associations et/ou étant éventuellement accolés à un cycle C₉ hydrocarboné de 5 à 6 atomes de carbone ; R₁ représente un radical alkyle en C₁-C₂₀ linéaire ou ramifié, saturé ou insaturé, éventuellement substitué par au moins un substituant A₁, un cycle hydrocarboné ou un hétérocycle Hy₂ éventuellement substitué par au moins un substituant A₃, C₈, C₉, A₁, Hy₂ et A₃ ont la signification donnée précédemment.

Par sels de composé de formule (I), on entend selon l'invention, les sels organiques ou inorganiques d'un composé de formule (I).

Comme sels inorganiques utilisables selon l'invention on peut citer : les sels de sodium ou de potassium ainsi que les sels de zinc (Zn²⁺), de calcium (Ca²⁺), de cuivre (Cu²⁺), de fer (Fe²⁺), de strontium (Sr²⁺), de magnésium (Mg²⁺) et de manganèse (Mn²⁺) ; les hydroxydes et les carbonates.

Les sels organiques utilisables selon l'invention sont par exemple les sels de tri-éthanolamine, mono-éthanolamine, di-éthanolamine, hexadécylamine et N,N,N',N'-tétrakis-(hydroxy-propyl-2) éthylène diamine, tris-hydroxyméthylaminométhane.

Comme exemple de composés 2-thioacétamide de formule (I) utilisable dans l'invention on peut citer les composés suivants :

### Composé 1 :

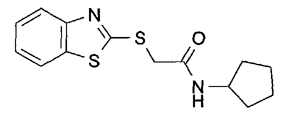

### Composé 2 :

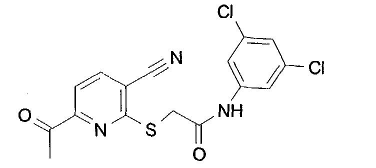

### Composé 3 :

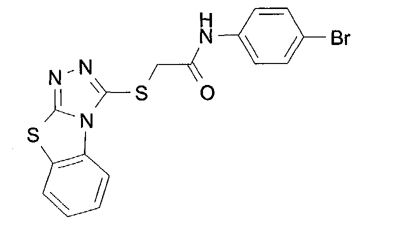

### Composé 4 :

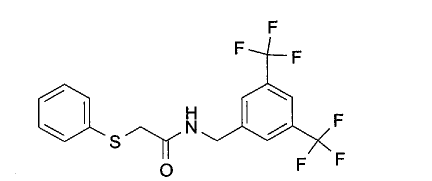

### Composé 5 :

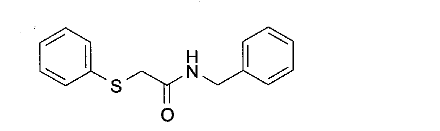

### Composé 6 :

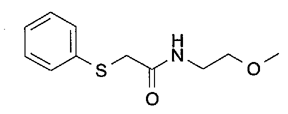

- CH₂-CH₂O-CH₃

### Composé 7 :

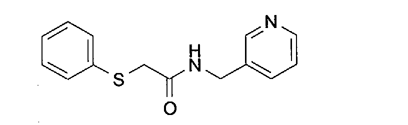

### Composé 8 :

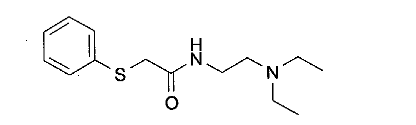

### Composé 9 :

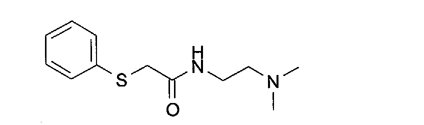

### Composé 10 :

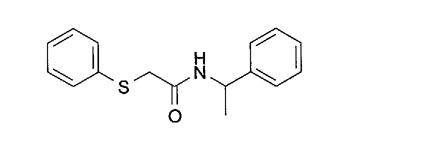

### Composé 11 :

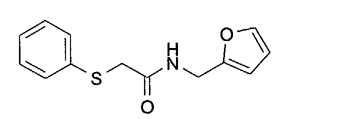

### Composé 12 :

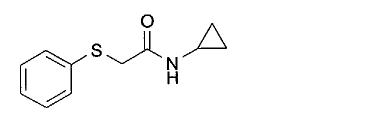

### Composé 13 :

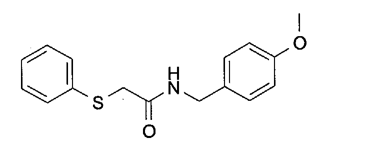

### Composé 14 :

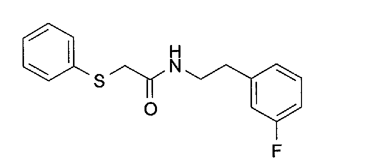

### Composé 15 :

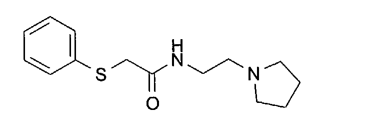

### Composé 16 :

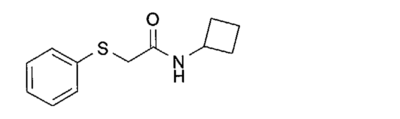

### Composé 17 :

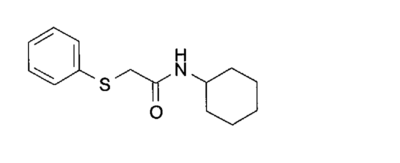

### Composé 18 :

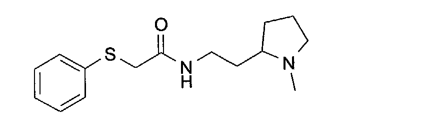

### Composé 19 :

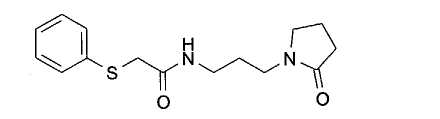

### Composé 20 :

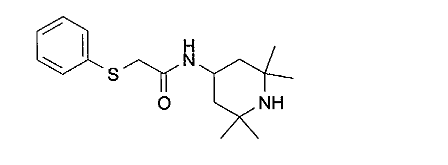

### Composé 21 :

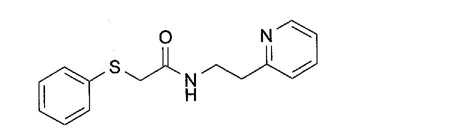

### Composé 22 :

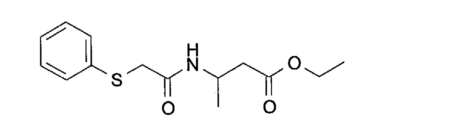

### Composé 23 :

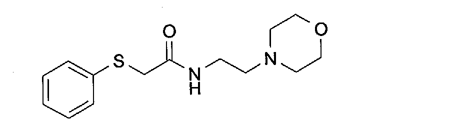

### Composé 24 :

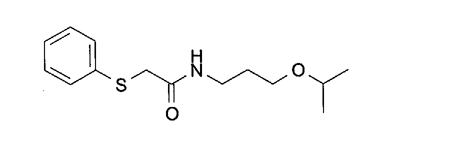

### Composé 25 :

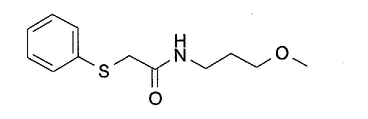

### Composé 26 :

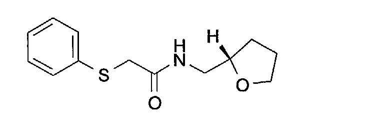

### Composé 27 :

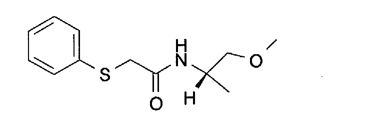

### Composé 28 :

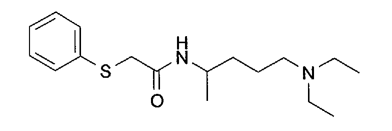

### Composé 29 :

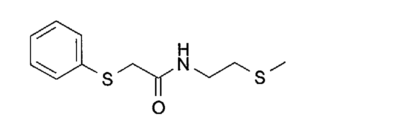

### Composé 30 :

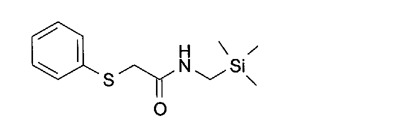

### Composé 31 :

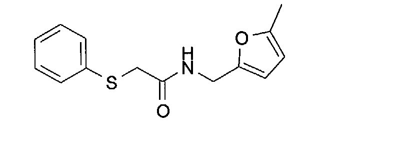

### Composé 32 :

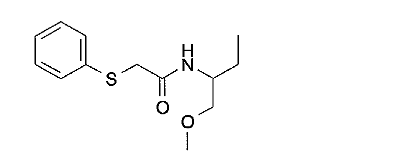

### Composé 33 :

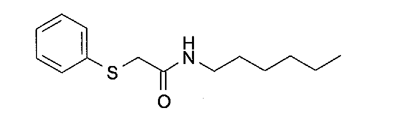

### Composé 34 :

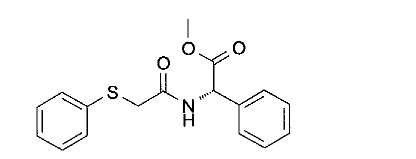

### Composé 35 :

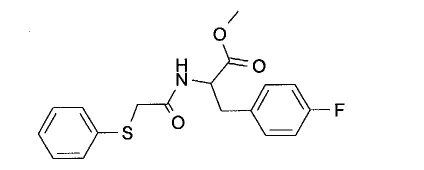

### Composé 36 :

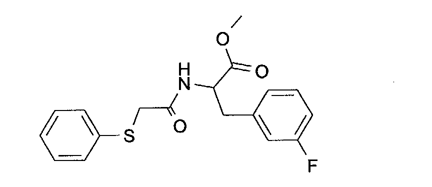

### Composé 37 :

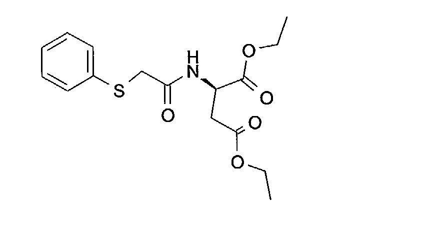

### Composé 38 :

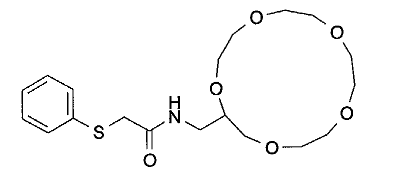

### Composé 39 :

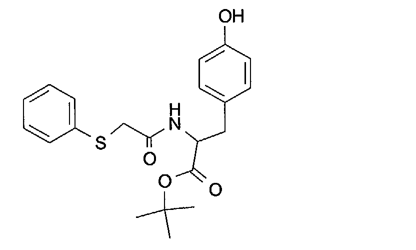

### Composé 40 :

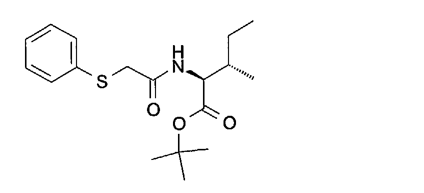

### Composé 41 :

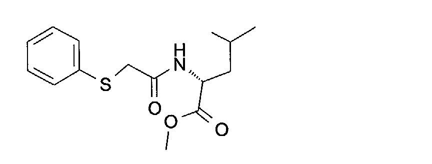

### Composé 42 :

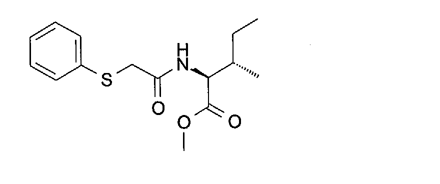

### Composé 43 :

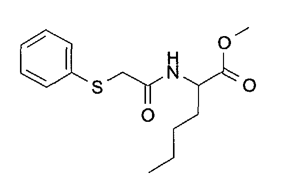

Les composés de formule (I), salifiés ou non peuvent être fabriqués de façon classique par condensation successive de deux molécules nucléophiles sur le chlorure de l'acide chloro-acétyle. Par exemple, la fonction amide est réalisée par condensation d'une amine sur le chlorure de chloro-acétyle en milieu basique. Le produit obtenu est lui-même condensé avec un thiolate.

A la connaissance du demandeur, aucun document de l'art antérieur ne décrit ni ne suggère que les composés 2-thioacétamide de formule (I) ou leurs sels ont la propriété d'induire et/ou de stimuler la croissance des fibres kératiniques humaines et en particulier des cheveux humains et des cils et/ou de freiner leur chute ni que ces composés peuvent être utilisés par voie topique pour augmenter la densité des kératiniques humaines et plus spécialement des cheveux et des cils.

La quantité efficace d'un composé de formule (I) ou de l'un de ses sels correspond à la quantité nécessaire pour obtenir le résultat désiré (à savoir augmenter la densité des fibres kératiniques et notamment des cheveux et des cils ou favoriser leur pousse). L'homme du métier est donc en mesure d'évaluer cette quantité efficace qui dépend de la nature du composé utilisé, de la personne à laquelle on l'applique, et du temps de cette application.

Dans la suite du texte, et sauf indication contraire, les quantités des différents ingrédients de la composition sont données en pourcentage en poids par rapport au poids total de la composition.

Pour donner un ordre de grandeur, selon l'invention, le composé de formule (I) ou l'un de ses sels ou un mélange de composés de formule (I) et/ou de leurs sels peut être utilisé en une quantité représentant de 10⁻³ % à 5% du poids total de la composition et préférentiellement en une quantité représentant de 10⁻² % à 2% du poids total de la composition, par exemple de 0,5 à 2 %.

La composition de l'invention peut être à usage cosmétique ou pharmaceutique. Préférentiellement, la composition de l'invention est à usage cosmétique. Aussi, la composition doit contenir un milieu physiologiquement acceptable non toxique et susceptible d'être appliqué sur la peau, y compris le cuir chevelu et les paupières, et sur les fibres kératiniques d'êtres humains. Par "cosmétique", on entend au sens de l'invention une composition d'aspect, d'odeur et de toucher agréables.

Le composé de formule (I), salifié ou non, peut être utilisé dans une composition qui doit être ingérée, injectée ou appliquée sur la peau ou sur les fibres kératiniques (sur toute zone cutanée ou des fibres à traiter).

Selon l'invention, le composé de formule (I) ou un mélange de composés de formule (I) peut être utilisé par la voie orale en une quantité de 0,1 à 300 mg par jour, 5 à 10mg/j.

Une composition préférée de l'invention est une composition à usage cosmétique et en particulier d'application topique sur la peau et les fibres kératiniques, et plus spécialement sur le cuir chevelu, les cheveux et les cils.

Cette composition peut se présenter sous toutes formes galéniques connues adaptées au mode d'utilisation.

Pour une application topique sur la peau ou les fibres kératiniques, la composition peut avoir la forme d'une solution ou suspension aqueuse, alcoolique ou hydro-alcoolique ou d'une suspension ou solution huileuse, d'une émulsion ou dispersion de consistance plus ou moins fluide et notamment liquide ou semi-liquide, obtenue par dispersion d'une phase grasse dans une phase aqueuse (H/E) ou inversement (E/H), d'une émulsion ou dispersion solide (H/E) ou (E/H), d'un gel aqueux, hydro-alcoolique ou huileux plus ou moins fluide ou solide, d'une poudre libre ou compactée à utiliser telle quelle ou à incorporer dans un milieu physiologiquement acceptable, ou encore de microcapsules ou microparticules, de dispersions vésiculaires de type ionique et/ou non ionique.

On peut également envisager une composition sous forme de mousse ou encore sous forme de spray ou d'aérosol comprenant alors un agent propulseur sous pression.

Elle peut ainsi se présenter sous forme d'une lotion, sérum, lait, crème H/E ou E/H, gel, onguent, pommade, poudre, baume, patch, tampon imbibé, pain, mousse.

En particulier la composition à application sur le cuir chevelu ou les cheveux peut se présenter sous forme d'une lotion de soin capillaire, par exemple d'application journalière ou bi-hebdomadaire, d'un shampooing ou d'un après-shampooing capillaire, en particulier d'application bi-hebdomadaire ou hebdomadaire, d'un savon liquide ou solide de nettoyage du cuir chevelu d'application journalière, d'un produit de mise en forme de la coiffure (laque, produit pour mise en pli, gel coiffant), d'un masque traitant, d'une crème ou d'un gel moussant de nettoyage des cheveux. Elle peut encore se présenter sous forme de teinture ou de mascara capillaire à appliquer au pinceau ou au peigne.

Par ailleurs, pour une application sur les cils ou les poils, la composition à laquelle s'applique l'invention peut se présenter sous forme d'un mascara, pigmenté ou non, à appliquer à la brosse sur les cils ou encore sur les poils de barbe ou de moustache.

Pour une composition à usage par injection, la composition peut se présenter sous forme de lotion aqueuse ou de suspension huileuse. Pour un usage par voie orale, la composition peut se présenter sous forme de capsules, de granulés, de sirops buvables ou de comprimés.

Selon un mode de réalisation particulier, la composition selon l'invention se présente sous forme de crème ou lotion capillaire, de shampooing ou d'après-shampooing capillaire, de mascara capillaire ou pour cils.

Les quantités des différents constituants du milieu physiologique de la composition selon l'invention sont celles généralement utilisées dans les domaines considérés. En outre, ces compositions sont préparées selon les méthodes usuelles.

Lorsque la composition est une émulsion, la proportion de la phase grasse peut aller de 2 % à 80 % en poids, et de préférence de 5 % à 50 % en poids par rapport au poids total de la composition. La phase aqueuse est ajustée en fonction de la teneur en phase grasse et en composé(s) (I) ainsi que de celle des éventuels ingrédients additionnels, pour obtenir 100% en poids. En pratique la phase aqueuse représente de 5 % à 99,9% en poids.

La phase grasse peut contenir des composés gras ou huileux, liquides à température ambiante (25°C) et pression atmosphérique (760 mm de Hg), généralement appelés huiles. Ces huiles peuvent être compatibles ou non entre elles et former une phase grasse liquide macroscopiquement homogène ou un système bi- ou triphasique.

La phase grasse peut, en plus des huiles, contenir des cires, des gommes, des polymères lipophiles, des produits "pâteux" ou visqueux contenant des parties solides et des parties liquides.

La phase aqueuse contient de l'eau et éventuellement un ingrédient miscible en toute proportion à l'eau comme les alcools inférieurs en C₁ à C₈ tel que l'éthanol, l'isopropanol, les polyols comme le propylène glycol, le glycérol, le sorbitol ou encore l'acétone ou l'éther.

Les émulsionnants et co-émulsionnants utilisés pour l'obtention d'une composition sous forme d'émulsion sont ceux généralement utilisés dans les domaines cosmétique et pharmaceutique. Leur nature est, en outre, fonction du sens de l'émulsion. En pratique, l'émulsionnant et éventuellement le co-émulsionnant sont présents, dans la composition, en une proportion allant de 0,1 % à 30 % en poids, de préférence de 0,5 à 20 % en poids et mieux de 1 à 8%. L'émulsion peut, en outre, contenir des vésicules lipidiques et notamment des liposomes.

Lorsque la composition est sous forme d'une solution ou d'un gel huileux, la phase grasse peut représenter plus de 90 % du poids total de la composition.

Avantageusement, pour une application capillaire, la composition de l'invention est une solution ou suspension aqueuse, alcoolique ou hydro-alcoolique et mieux une solution ou suspension eau/éthanol. La fraction alcoolique peut représenter de 5% à 99,9% et mieux de 8% à 80%.

Pour une application mascara, la composition de l'invention est notamment sous forme d'une dispersion de cire-dans-eau ou de cire-dans-huile, d'une huile gélifiée, d'un gel aqueux, pigmenté ou non.

La composition de l'invention peut comprendre, en outre, d'autres ingrédients additionnels usuellement utilisés dans les domaines concernés, choisis parmi les solvants, les épaississants ou gélifiants de phase aqueuse ou de phase huileuse, les matières colorantes solubles dans le milieu de la composition, les particules solides du type charges ou pigments, les antioxydants, les conservateurs, les parfums, les électrolytes, les neutralisants, les polymères filmogènes, les agents bloqueurs d'U.V. comme les filtres solaires, les actifs cosmétiques et pharmaceutiques à action bénéfique pour la peau ou les fibres kératiniques, autres que les composés de formule (I), leurs mélanges. Ces additifs peuvent être présents dans la composition selon les quantités généralement utilisées dans le domaine cosmétique et dermatologique et notamment à raison de 0,01 à 50% du poids total de la composition et mieux de 0,1 à 20% et par exemple de 0,1 à 10 %. Ces additifs selon leur nature, peuvent être introduits dans la phase grasse, dans la phase aqueuse et/ou dans les vésicules lipidiques et notamment des liposomes.

Bien entendu l'homme du métier veillera à choisir les éventuels ingrédients additionnels et/ou leur quantité de telle manière que les propriétés avantageuses de la composition selon l'invention, à savoir l'inhibition de la 15-PGDH et notamment l'augmentation de la densité des fibres kératiniques, ne soient pas ou substantiellement pas, altérées par l'adjonction envisagée.

Comme solvants utilisables dans l'invention, on peut citer les alcools inférieurs en C₂ à C₈ comme l'éthanol, l'isopropanol, le propylène glycol et certaines huiles cosmétiques légères comme les alcanes en C₆ à C₁₆.

Comme huiles utilisables dans l'invention, on peut citer les huiles d'origine minérale (huile de vaseline, isoparaffine hydrogénée), les huiles d'origine végétale (fraction liquide du beurre de karité, huile de tournesol, d'abricot, alcool ou acide gras), les huiles d'origine animale (perhydrosqualène), les huiles de synthèse (ester d'acide gras, huile de Purcellin), les huiles siliconées (polydiméthylsiloxane linéaire ou cyclique, phényltriméthicone) et les huiles fluorées (perfluoropolyéthers). Comme cires, on peut citer les cires siliconées, les cires d'abeille, de riz, de candellila, de carnauba ou paraffine, de polyéthylène.

Comme émulsionnants utilisables dans l'invention, on peut citer par exemple le stéarate ou laurate de glycérol, les stéarates ou oléates de sorbitol, les alkyl diméthiconecopolyol (avec alkyle ≥ 8) et leurs mélanges pour une émulsion E/H. On peut aussi utiliser le monostéarate ou monolaurate de polyéthylène glycol, le stéarate ou oléate de sorbitol polyoxyéthyléné, les diméthiconecopolyols et leurs mélanges pour une émulsion H/E.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les Bentones, les sels métalliques d'acides gras comme les stéarates d'aluminium, la silice traitée hydrophobe, l'éthylcellulose, leurs mélanges.

Comme actif cosmétique ou pharmaceutique, autre que le composé de formule (I) utilisable dans l'invention, on peut citer les actifs hydrophiles comme les protéines ou hydrolysats de protéine, les acides aminés, les polyols, l'urée, l'allantoïne, les sucres et les dérivés de sucre, les vitamines hydrosolubles, les extraits végétaux (ceux d'Iridacées ou de soja) et les hydroxy-acides comme les acides de fruits ou l'acide salicylique ; et les actifs lipophiles comme le rétinol (vitamine A) et ses dérivés notamment ester (palmitate de rétinol), le tocophérol (vitamine E) et ses dérivés notamment ester (acétate de tocophérol), les acides gras essentiels, les céramides, les huiles essentielles, les dérivés de l'acide salicylique comme le n-octanoyl-5 salicylique, les esters des hydroxy-acides, les phospholipides comme la lécithine, leurs mélanges.

Selon un mode particulier de réalisation de l'invention, on peut associer au composé de formule (I), au moins un composé actif additionnel favorisant la repousse et/ou limitant la chute des fibres kératiniques (cheveux, cils). Ces composés additionnels sont notamment choisis parmi les inhibiteurs de lipoxygénase tels que décrits dans EP 0648488, les inhibiteurs de bradykinine décrits notamment dans EP 0845700, les prostaglandines et leurs dérivés notamment ceux décrits dans WO 98/33497, WO 95/11003, JP 97-100091, JP 96-134242, les agonistes ou antagonistes des récepteurs des prostaglandines, les analogues non prostanoïques de prostaglandines tels que décrits dans EP 1175891 et EP 1175890, WO 01/74307, WO 01/74313, WO 01/74314, WO 01/74315 ou WO 01/72268, leurs mélanges.

Comme autres composés actifs additionnels favorisant la pousse des fibres kératiniques et/ou limitant leur chute pouvant être présents dans la composition selon l'invention, on peut citer les vasodilatateurs, les antiandrogènes, les cyclosporines et leurs analogues, les antimicrobiens et antifongiques, les anti-inflammatoires, les rétinoïdes, seuls ou en mélange.

Les vasodilatateurs utilisables sont notamment les agonistes des canaux potassium incluant le minoxidil ainsi que les composés décrits dans les brevets US 3 382247, 5 756092, 5 772990, 5 760043, 5 466694, 5 438058, 4 973474, la cromakalim, le nicorandil et le diaxozide, seuls ou en association.

Les anti-androgènes utilisables incluent notamment les inhibiteurs stéroïdiens ou non stéroïdiens de 5α-réductase, comme le finastéride et les composés décrits dans US 5 516779, l'acétate de cyprostérone, l'acide azélaïque, ses sels et ses dérivés et les composés décrits dans US 5 480913, le flutamide, l'oxendolone, la spironolactone, le diéthylstilbestrol et les composés décrits dans les brevets US 5 411981, 5 565467 et 4 910226.

Les composés antimicrobiens ou antifongiques peuvent être choisis parmi les dérivés du sélénium, l'octopirox, le triclocarban, le triclosan, le pyrithione zinc, l'itraconazole, l'acide asiatique, l'hinokitiol, la mipirocine, les tétracyclines, notamment l'érythromycine et les composés décrits dans EP 0680745, le chlorhydrate de clinycine, le peroxyde de benzoyle ou de benzyle, la minocycline et les composés appartenant à la classe des imidazoles tels que l'éconazole, le kétoconazole ou le miconazole ou leurs sels, les esters d'acide nicotinique, dont notamment le nicotinate de tocophérol, le nicotinate de benzyle et les nicotinates d'alkyles en C₁-C₆ comme les nicotinates de méthyle ou d'hexyle.

Les anti-inflammatoires peuvent être choisis parmi les anti-inflammatoires stéroïdiens comme les glucocorticoïdes, les corticostéroïdes (par exemple : l'hydrocortisone) et les anti-inflammatoires non stéroïdiens comme l'acide glycyrrhétinique et l'α-bisabolol, la benzydamine, l'acide salicylique et les composés décrits dans EP 0770399, WO 94/06434 et FR 2268523.

Les rétinoïdes peuvent être choisis parmi l'isotrétinoïne, l'acitrétine et le tazarotène.

Comme autres composés actifs pour favoriser la pousse et/ou limiter la chute des cheveux utilisables en associations avec le composé de formule (I), salifié ou non, on peut citer l'aminexil, le 6-0-[(9Z,12Z)-octadéca-9,12-diènoyl]hexapyranose, le chlorure de benzalkonium, le chlorure de benzéthonium, le phénol, l'oestradiol, le maléate de chlorphéniramine, les dérivés de chlorophylline, le cholestérol, la cystéine, la méthionine, le menthol, l'huile de menthe poivrée, le panthoténate de calcium, le panthénol, le résorcinol, les activateurs de la protéine kinase C, les inhibiteurs de la glycosidase, les inhibiteurs de glycosaminoglycanase, les esters d'acide pyroglutamique, les acides hexosaccharidiques ou acyl-hexosaccharique, les éthylènes aryle substitués, les amino-acides N-acylés, les flavonoïdes, les dérivés et analogues d'ascomycine, les antagonistes d'histamine, les saponines, les inhibiteurs de protéoglycanase, les agonistes et antagonistes d'estrogènes, les pseudotérines, les cytokines et les promoteurs de facteurs de croissance, les inhibiteurs d'IL-1 ou d'IL-6, les promoteurs d'IL-10, les inhibiteurs de TNF, les benzophénones et l'hydantoïne, l'acide rétinoïque ; les vitamines comme la vitamine D, les analogues de la vitamine B12 et le panthoténol ; les triterpènes comme l'acide ursolique et les composés décrits dans US 5529769, US 5468888, US 5631282 ; les agents antiprurigineux comme la thénaldine, la triméprazine ou la cyproheptadine ; les antiparasitaires, en particulier le métronidazole, le crotamiton ou les pyréthrinoïdes ; les agents antagonistes de calcium, comme la cinnarizine, le diltiazem, la nimodipine, vérapamil, l'alvérine et la nifédipine ; les hormones telles que l'estriol ou ses analogues, la thyroxine et ses sels, la progestérone ; les antagonistes du récepteur FP (récepteur aux prostaglandines du type F) tels que le latanoprost, le bimatoprost, le travoprost, l'unoprostone ; leurs mélanges.

Avantageusement, la composition selon l'invention comprend au moins un inhibiteur de la 15-PGDH tel que défini précédemment et au moins une prostaglandine ou un dérivé de prostaglandine comme par exemple les prostaglandines de la série 2 dont notamment PGF2-α et PGE2 sous forme saline ou ester (exemple les isopropyl esters), leurs dérivés comme le 16,16 diméthyl PGE2, le 17 phényl PGE2, le 16,16 diméthyl PGF2-α, le 17 phényl PGF2-α les prostaglandines de la série 1 comme le 11 déoxy prostaglandine E1, le 1 déoxy prostaglandine E1 sous forme saline ou ester, leurs analogues notamment le latanoprost, le fluprosténol, le bimatoprost, le cloprosténol, le viprostol, le butaprost, le misoprostol, l'unoprostone, leurs sels ou leurs esters.

De manière avantageuse, la composition contient au moins un agoniste non prostanoïque des récepteurs EP2 et/ou EP4 notamment tel que décrit dans EP 1175892.

On peut également envisager que la composition comprenant au moins le composé de formule (I), salifié ou non soit sous forme liposomée, telle que notamment décrite dans le document WO 94/22468. Ainsi, le composé encapsulé dans les liposomes peut être délivré sélectivement au niveau du follicule pileux.

La composition selon l'invention peut être appliquée sur les zones alopéciques du cuir chevelu et des cheveux d'un individu, et éventuellement laissée en contact plusieurs heures et éventuellement rincée.

On peut, par exemple, appliquer la composition contenant une quantité efficace d'un composé de formule (I), salifié ou non, le soir, garder celle-ci au contact toute la nuit et éventuellement effectuer un shampooing le matin. Ces applications peuvent être renouvelées quotidiennement pendant un ou plusieurs mois suivant les individus.

Ainsi, la présente invention a également pour objet un procédé de traitement cosmétique des fibres kératiniques humaines et/ou de la peau d'où émergent lesdites fibres, y compris du cuir chevelu et des paupières, caractérisé par le fait qu'il consiste à appliquer sur les fibres kératiniques et/ou la peau d'où émergent lesdites fibres, une composition cosmétique comprenant au moins un dérivé de composé (I) ou l'un de ses sels, à laisser celle-ci en contact avec les fibres kératiniques et/ou la peau d'où émergent lesdites fibres, et éventuellement à rincer les fibres et/ou la peau.

Ce procédé de traitement présente bien les caractéristiques d'un procédé cosmétique dans la mesure où il permet d'améliorer l'esthétique des fibres kératiniques humaines en leur donnant une plus grande vigueur et un aspect amélioré. En outre, il peut être utilisé quotidiennement pendant plusieurs mois, sans prescription médicale.

Plus spécialement, la présente invention a pour objet un procédé de soin cosmétique des cheveux et/ou du cuir chevelu humains, en vue d'améliorer leur état et/ou leur aspect, caractérisé en ce qu'il consiste à appliquer sur les cheveux et/ou le cuir chevelu, une composition cosmétique comprenant une quantité efficace d'au moins un composé de formule (I) ou l'un de ses sels, à laisser celle-ci en contact avec les cheveux et/ou le cuir chevelu, et éventuellement à rincer les cheveux et/ou le cuir chevelu.

Avantageusement, dans le procédé selon l'invention, on applique sur les zones à traiter du cuir chevelu entre 5 et 500 µl d'une solution ou composition telle que définie précédemment, comprenant de 0,001% à 5 % d'inhibiteur de la 15-PGDH.

L'invention a encore pour objet un procédé de soin cosmétique et/ou de maquillage des cils humains, en vue d'améliorer leur état et/ou leur aspect, caractérisé en ce qu'il consiste à appliquer sur les cils et/ou les paupières une composition de mascara comprenant au moins un composé de formule (I) ou l'un de ses sels et à laisser celle-ci au contact des cils et/ou les paupières. Cette composition de mascara peut être appliquée seule ou en sous-couche d'un mascara pigmenté classique et être éliminée comme un mascara pigmenté classique.

L'invention a encore pour objet une composition de soin ou de maquillage des fibres kératiniques, comprenant dans un milieu physiologiquement acceptable, en particulier cosmétique, au moins un dérivé de formule (I) ou l'un de ses sels et au moins un actif additionnel favorisant la repousse des fibres kératiniques humaines et/ou limitant la chute choisi parmi l'aminexil, les agonistes du récepteur FP et les vasodilatateurs et plus spécialement choisi parmi l'aminexil, le minoxidil, le latanoprost et le travoprost.

On va maintenant donner à titre d'illustration des exemples de réalisation de l'invention qui ne sauraient limiter en aucune façon sa portée.

### Exemple 1 : Schéma réactionnel de la synthèse du composé 1 :

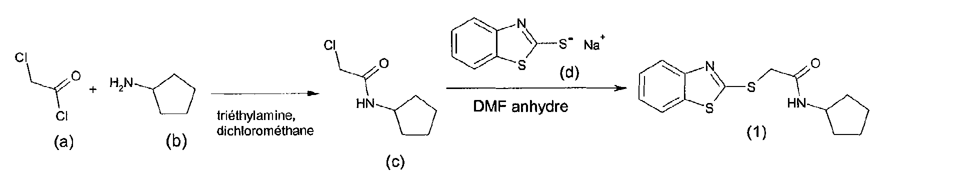

### Mode Opératoire

Dans un tricol de 250 mL muni d'un réfrigérant, d'un thermomètre, d'une ampoule à addition ainsi que d'une agitation magnétique, la cyclopentylamine (composé b) (11,65 mL, 0,117 mol) est diluée dans 100 mL de dichlorométhane. La triéthylamine (18,15 mL, 0,129 mol) est ajoutée, puis le milieu est refroidit à 10°C. Le chlorure de l'acide chloro-acétyle (composé a) (9,35 mL, 0,117 mol) préalablement dilué dans 25 mL de dichlorométhane est alors ajouté goutte à goutte tout en conservant une température entre 10°C et 15°C. Après cette addition, le milieu réactionnel est agité à température ambiante pendant 1 heure. Le mélange est ensuite lavé avec de l'eau (2 fois 100 mL), une solution d'acide chlorhydrique 1N (2 fois 50 mL), puis avec de l'eau (2 fois 100 mL) et enfin avec une solution de chlorure de sodium saturée. La phase organique est séchée sur du sulfate de sodium, filtrée puis concentrée au maximum. On obtient 15,5 g d'un solide marron (composé c) avec un rendement de 82%.

Dans un tricol de 250 mL muni d'un réfrigérant, d'un thermomètre, d'une entrée d'argon ainsi que d'une agitation magnétique, le solide marron (8,51 g, 53 mmol) obtenu précédemment est dissout dans 100 mL de diméthylformamide (DMF) anhydre puis on additionne le sel de sodium du 2-mercaptobenzothiazole (composé d) (10 g, 53 mmol). Le milieu réactionnel est chauffé à 60°C pendant 5 heures puis concentré au maximum. Le mélange est ensuite dilué avec 100 mL de dichlorométhane puis lavé avec de l'eau (3 fois 100 mL), une solution d'acide chlorhydrique 2N (1 fois 50 mL), puis avec de l'eau (2 fois 100 mL) et enfin avec une solution de chlorure de sodium saturée. La phase organique est séchée sur du sulfate de sodium, filtrée puis concentrée au maximum pour donner un solide marron. Ce dernier est repris dans 200 mL d'éther diéthylique et laissé sous agitation à température ambiante pendant 2 heures. Le solide obtenu est filtré sur fritté puis séché sous vide en présence de pentoxyde de phosphore. Le produit 1 est obtenu sous forme d'un solide de couleur beige (11,5 g) avec un rendement de 74%.

**Spectrométrie de masse** : Les ions quasi-moléculaires (MH)+, (MNa)+ et (M-H)- de la molécule attendue, C₁₄H₁₆N₂OS₂, sont principalement détectés

**Résonance Magnétique Nucléaire** : Les spectres obtenus sont en accord avec la structure proposée ; dédoublement de certains signaux dû au forme syn et anti de l'amide. Le dédoublement disparaît en chauffant. RMN ¹H (CDCl₃) δ : 1,38 (m, 2H) ; 1,54 (m, 4H) ; 1,88 (m, 2H) ; 3,89 (s, 2H) ; 4,17 (m, 1 H) ; 7,33 (m, 1 H) ; 7,44 (m, 1 H) ; 7,53 (se, 1 H) ; 7,76 (d, 1 H) ; 7,82 (d, 1 H).

### Exemples 2 et 3 :

Les composés 2 et 3 peuvent être synthétisés par une méthode de synthèse analogue à celle du composé 1.

### Exemples 4 à 43 : Schéma réactionnel de la synthèse des composés 4 à 43 :

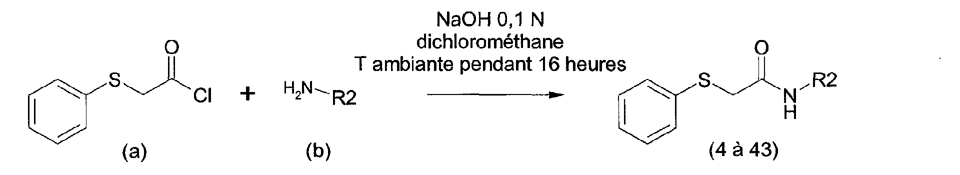

A 0,02mmol du composé (a) en solution dans 500µL de dichlorométhane est ajouté une solution de 0,022 mmol d'amine dans 500µL de dichlorométhane. 1 ml d'une solution de soude 0,1N est ajouté au milieu réactionnel et l'agitation est poursuivie pendant une nuit à température ambiante. Le milieu réactionnel est séché par passage sur cartouche Chem Elut®(Varian), rincée par 5 mL de dichlorométhane. Le solvant est évaporé. L'amide est obtenu sous forme d'un solide (rendement 40-100%).

### Exemple 44 : Mise en évidence des propriétés inhibitrices spécifiques de la 15-PGDH des composés de formule (I).

### 1°) Test sur 15-PGDH

L'enzyme 15-PGDH est obtenue comme décrit dans la demande FR 02/05067 déposée au nom de L'Oréal, en suspension dans un milieu adapté à une concentration de 0,3 mg/mL puis bloquée à - 80°C. Pour les besoins du test, cette suspension est décongelée et stockée dans de la glace.

Par ailleurs on prépare un tampon Tris 100 mM, pH = 7,4, contenant 0,1 mM de dithiothréitol (D5545, Sigma-Aldrich, L'isle D'Abeau Chesne, BP 701, 38297, Saint Quentin Fallavier), 1,5 mM de β-NAD (N6522, Sigma-Aldrich, L'isle D'Abeau Chesne, BP 701, 38297, Saint Quentin Fallavier), 50 µM de Prostaglandine E₂ (P4172, Sigma-Aldrich, L'isle D'Abeau Chesne, BP 701, 38297, Saint Quentin Fallavier).

Dans la cuve d'un spectrophotomètre (Perkin-Elmer, Lambda 2) thermostaté à 37°C, dont la longueur d'onde de mesure est réglée à 340 nm, sont introduits 0,965 ml de ce tampon (préalablement porté à 37°C). 0,035 mL de suspension enzymatique à 37°C sont introduits dans la cuve concomitamment à l'enregistrement ( correspondant à une augmentation de la densité optique à 340 nm). La vitesse maximale de réaction est relevée.

Les valeurs essais (contenant les composés (I)) sont comparées à la valeur témoin (sans composé (I)) ; les résultats indiqués représentent la concentration à laquelle le composé de formule (I) réduit de 50% l'activité enzymatique de 15-PGDH, à savoir IC_{50dh}.

### 2°) Test sur PGF Synthase

L'enzyme PGFS est obtenue comme décrit dans le document FR-A-02/05067, à la concentration de 0,5 mg/mL, en suspension dans un milieu approprié, et bloquée à - 80°C. Pour les besoins du test, cette suspension est décongelée et stockée dans la glace.

Par ailleurs, on prépare dans un flacon brun (abri de la lumière) un tampon Tris 100, mM, pH = 6,5 contenant 20 µM de 9,10 phénanthrène quinone* (P2896, Sigma-Aldrich, L'isle D'Abeau Chesne, BP 701, 38297, Saint Quentin Fallavier) et 100 µM de β-NADPH (N1630, Sigma-Aldrich, L'isle D'Abeau Chesne, BP 701, 38297, Saint Quentin Fallavier).
* Une solution mère titrant 1 mM est préparée dans de l'éthanol absolu, portée à 40°C ; le flacon est placé dans une cuve à ultrason pour faciliter la solubilisation du produit.

Dans la cuve d'un spectrophotomètre (Perkin-Elmer, Lambda 2) thermostaté à 37°C, dont la longueur d'onde de mesure est réglée à 340 nm sont introduits 0,950 mL de ce tampon (préalablement porté à 37°C). 0,05 mL de suspension enzymatique à 37°C sont introduits dans la cuve concomitamment à l'enregistrement (correspondant à une baisse de la densité optique à 340 nm). La vitesse maximale de réaction est relevée.

Les valeurs essais (contenant le composé (I)) sont comparées à la valeur témoin (sans composé (I)) ; les résultats indiqués représentent la concentration à laquelle le composé de formule (I) réduit de 50% l'activité enzymatique de PGFS, à savoir IC_{50fs}.

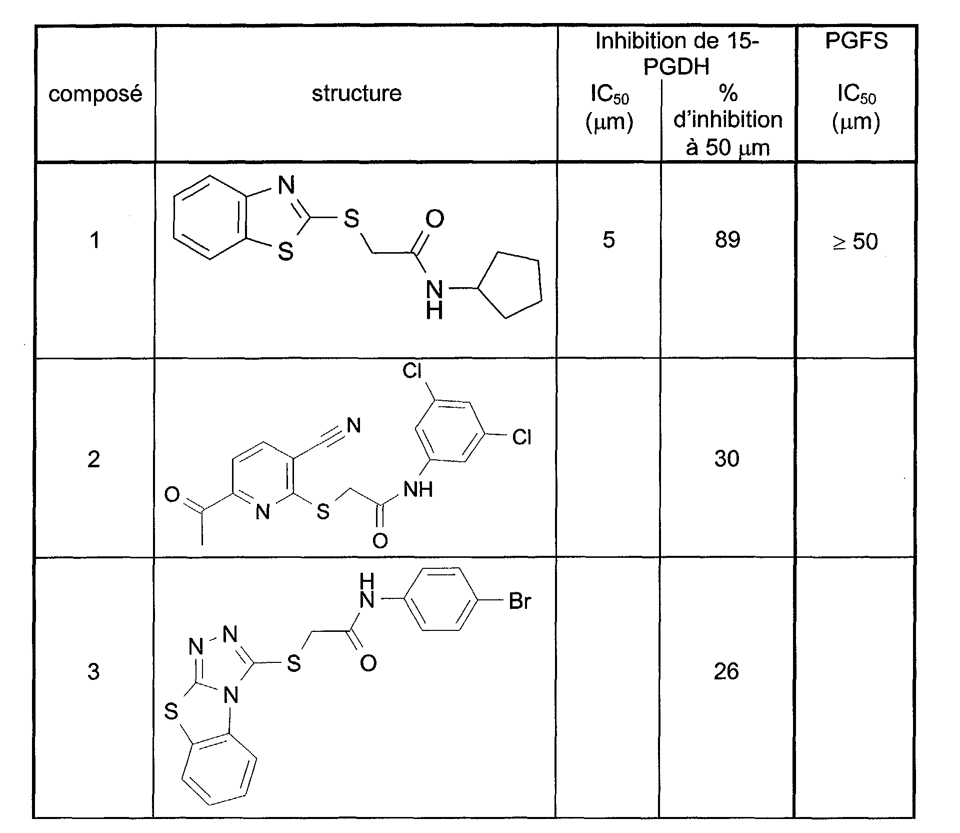

De ce tableau, il ressort que le composé 1 est bien un inhibiteur de 15-PGDH. De plus, il inhibe de façon plus efficace et sélective la 15-PGDH que la PGFS. Ainsi le rapport IC_{50fs}/IC_{50dh} est supérieur à 10.

Les compositions ci-après sont obtenues par les techniques habituelles couramment utilisées dans le domaine cosmétique ou pharmaceutique.

Dans chacun des exemples de formule, on obtient un fluide qui peut être utilisé en application mono- ou bi-quotidienne sur le cuir chevelu, pour diminuer la chute et/ou favoriser la pousse des cheveux.

### EXEMPLE 45 : Lotion capillaire

- Composé de l'Exemple 1 1,00 g
- Propylène glycol 30,00 g
- Alcool éthylique 40,00 g
- Eau qsp 100,00 g

On applique cette lotion sur le cuir chevelu, une à deux fois par jour, à raison d'1 mL par application.

### EXEMPLE 46 : Lotion capillaire

- Composé 1 1,00 g
- Propylène glycol 30,00 g
- Alcool éthylique 40,00 g
- Eau qsp 100,00 g

On applique cette lotion sur le cuir chevelu, une à deux fois par jour, à raison d'1 mL par application, en massant légèrement le cuir chevelu pour faire pénétrer l'actif. La chevelure est ensuite séchée à l'air libre. Cette lotion permet de diminuer la chute des cheveux et de favoriser leur repousse.

### EXEMPLE 47 : Mascara cire/eau

- Cire d'abeilles 6,00 %
- Cire de paraffine 13,00 %
- Huile de jojoba hydrogénée 2 %
- Polymère filmogène hydrosoluble 3 %
- Stéarate de triéthanolamine 8 %
- Composé 1 %
- Pigment noir 5 %
- Conservateur qs
- Eau qsp 100 %

Ce mascara s'applique sur les cils comme un mascara classique avec une brosse à mascara.

## Revendications

1. Utilisation d'une quantité efficace d'au moins un composé 2-thioacétamide de formule (I) ou d'un de ses sels, 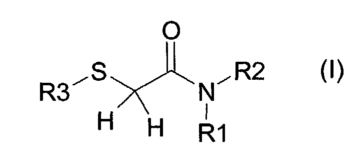 dans laquelle :
**a) R**_{**1**} **et R**_{**2**} représentent indépendamment :
1) un atome d'hydrogène,
2) un radical alkyle en C₁-C₂₀ éventuellement substitué par au moins un substituant A₁,
3) un cycle hydrocarboné C₁, éventuellement accolé à un cycle C₂ contenant éventuellement au moins un hétéroatome pour former un hétérocycle Hy₁, ces cycles C₁ et C₂ pouvant comporter au moins une fonction carbonyle ou thiocarbonyle et être substitués par au moins un substituant A₃,
4) un hétérocycle Hy₂ éventuellement accolé à un cycle C₂ contenant éventuellement au moins un hétéroatome pour former un hétérocycle Hy₃, ces cycles Hy₂ et C₂ pouvant comporter une fonction carbonyle ou thiocarbonyle et être substitués par au moins un substituant A₃, ou
5) un groupe C(=NR)R', C(=NR)NR'R", COR, CSR, COOR, CONRR', SO₂R ou SO₂NRR' ;
**b) R**_{**3**} représente :
1) un radical alkyle en C₁-C₂₀ éventuellement substitué par au moins un substituant A₂,
2) un cycle hydrocarboné C₃, éventuellement accolé à un cycle C₄ contenant éventuellement au moins un hétéroatome pour former un hétérocycle Hy₃, ces cycles C₃ et C₄ pouvant comporter au moins une fonction carbonyle ou thiocarbonyle et être substitués par au moins un substituant A₃,
3) un hétérocycle Hy₄ représentant un cycle pyrrole, furanne, thiophène ou pyrazole, éventuellement accolé à un cycle C₅ représentant un cycle phényle, pyridine ou pyrimidine, ces deux cycles Hy₄ et C₅ pouvant être substitués par au moins un substituant A₃,
4) un cycle pyridine éventuellement accolé à un cycle C₂ contenant éventuellement au moins un hétéroatome pour former un hétérocycle Hy₁, ces cycles pyridine et C₂ pouvant comporter au moins une fonction carbonyle ou thiocarbonyle et être substitués par au moins un substituant A₄,
5) un hétérocycle Hy₅ différent de Hy₄ et du cycle pyridine, éventuellement accolé à un cycle C₂ contenant éventuellement au moins un hétéroatome pour former un hétérocycle Hy₁, ces cycles Hy₅ et C₂ pouvant comporter au moins une fonction carbonyle ou thiocarbonyle et être substitués par au moins un substituant A₃, ou
6) un groupe C(=NR)R', C(=NR)NR'R", COR, CSR, COOR ou CONRR' ;
**c) A**_{**1**} représente :
1) un halogène,
2) un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R",
3) un cycle hydrocarboné C₆, éventuellement accolé à un cycle C₇ contenant éventuellement au moins un hétéroatome pour former un hétérocycle Hy₆, ces cycles C₆ et C₇ pouvant comporter au moins une fonction carbonyle ou thiocarbonyle et être substitués par au moins un substituant A₅,
4) un hétérocycle Hy₇ éventuellement accolé à un cycle C₇ contenant éventuellement au moins un hétéroatome pour former un hétérocycle Hy₆, ces cycles Hy₇ et C₇ pouvant comporter au moins une fonction carbonyle ou thiocarbonyle et être substitués par au moins un substituant A₅ ;
**d) A**_{**2**} représente :
1) un halogène,
2) un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R", ou
3) un cycle C₈ éventuellement accolé à un cycle C₉, ces cycles C₈ et C₉ contenant éventuellement au moins un hétéroatome pour former un hétérocycle Hy₈ et/ou au moins une fonction carbonyle ou thiocarbonyle et/ou étant substitués par au moins un substituant A₅ ;
**e) A**_{**3**} représente :
1) A₂ , ou
2) un groupe alkyle en C₁-C₂₀ éventuellement substitué par au moins un substituant A₅ ;
**f) A**_{**4**} représente :
1) un halogène,
2) un groupe CF₃, CN, OR, SR, NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R",
3) un groupe alkyle en C₁-C₂₀ éventuellement substitué par au moins un substituant A₅,
4) un cycle C₈ éventuellement accolé à un cycle C₉, ces cycles C₈ et C₉ contenant éventuellement au moins un hétéroatome pour former un hétérocycle Hy₈ et/ou au moins une fonction carbonyle ou thiocarbonyle et/ou étant substitués par au moins un substituant A₅ ;
**g) A**_{**5**} représente :
1) un halogène,
2) un groupe CF₃, CN, OR₅, SR₅, NR₅R₅', NR₅C(=NR₅')NR₅"R₅"', COR₅, CSR₅, COOR₅, CONR₅R₅', CSNR₅R₅', NR₅CSR₅', NR₅CSNR₅'R₅", NR₅COR₅', NR₅CONR₅'R₅", SO₂NR₅R₅', NR₅SO₂R₅', SO₂R₅ ou SiR₅R₅'R₅", R₅, R₅, R₅" étant un atome d'hydrogène ou un groupe alkyle en C₁-C₂₀,
3) un groupe alkyle en C₁-C₂₀, ou
4) un cycle C₁₀ éventuellement accolé à un autre cycle C₁₁, ces cycles contenant éventuellement au moins un hétéroatome pour former un hétérocycle Hy₉ et/ou comportant au moins une fonction carbonyle ou thiocarbonyle ;
**h) R, R', R" et R"'**, identiques ou différents, représentent :
1) un atome d'hydrogène,
2) un groupe alkyle en C₁-C₂₀ éventuellement substitué par au moins un substituant A₅, ou
3) un cycle C₁₂ éventuellement accolé à un autre cycle C₁₃, ces cycles contenant éventuellement au moins un hétéroatome pour former un hétérocycle Hy₁₀ et pouvant être substitués par au moins un substituant A₅ ;
**i) Hy**_{**1**} **à Hy**_{**10**} représentent indépendamment un hétérocycle pouvant contenir de 1 à 4 hétéroatomes choisis parmi N, O et S, comme agent pour induire et/ou stimuler la croissance des fibres kératiniques humaines et/ou freiner leur chute et/ou augmenter leur densité.

2. Utilisation cosmétique d'au moins un composé 2-thioacétamide de formule (I) ou d'un de ses sels, 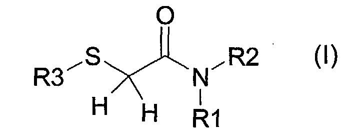 dans laquelle :
**a) R**_{**1**} **et R**_{**2**} représentent indépendamment :
1) un atome d'hydrogène,
2) un radical alkyle en C₁-C₂₀ éventuellement substitué par au moins un substituant A₁,
3) un cycle hydrocarboné C₁, éventuellement accolé à un cycle C₂ contenant éventuellement au moins un hétéroatome pour former un hétérocycle Hy₁, ces cycles C₁ et C₂ pouvant comporter au moins une fonction carbonyle ou thiocarbonyle et être substitués par au moins un substituant A₃,
4) un hétérocycle Hy₂ éventuellement accolé à un cycle C₂ contenant éventuellement au moins un hétéroatome pour former un hétérocycle Hy₃, ces cycles Hy₂ et C₂ pouvant comporter une fonction carbonyle ou thiocarbonyle et être substitués par au moins un substituant A₃, ou
5) un groupe C(=NR)R', C(=NR)NR'R", COR, CSR, COOR, CONRR', SO₂R ou SO₂NRR' ;
**b) R**_{**3**} représente :
1) un radical alkyle en C₁-C₂₀ éventuellement substitué par au moins un substituant A₂,
2) un cycle hydrocarboné C₃, éventuellement accolé à un cycle C₄ contenant éventuellement au moins un hétéroatome pour former un hétérocycle Hy₃, ces cycles C₃ et C₄ pouvant comporter au moins une fonction carbonyle ou thiocarbonyle et être substitués par au moins un substituant A₃,
3) un hétérocycle Hy₄ représentant un cycle pyrrole, furanne, thiophène ou pyrazole, éventuellement accolé à un cycle C₅ représentant un cycle phényle, pyridine ou pyrimidine, ces deux cycles Hy₄ et C₅ pouvant être substitués par au moins un substituant A₃,
4) un cycle pyridine éventuellement accolé à un cycle C₂ contenant éventuellement au moins un hétéroatome pour former un hétérocycle Hy₁, ces cycles pyridine et C₂ pouvant comporter au moins une fonction carbonyle ou thiocarbonyle et être substitués par au moins un substituant A₄,
5) un hétérocycle Hy₅ différent de Hy₄ et du cycle pyridine, éventuellement accolé à un cycle C₂ contenant éventuellement au moins un hétéroatome pour former un hétérocycle Hy₁, ces cycles Hy₅ et C₂ pouvant comporter au moins une fonction carbonyle ou thiocarbonyle et être substitués par au moins un substituant A₃, ou
6) un groupe C(=NR)R', C(=NR)NR'R", COR, CSR, COOR ou CONRR' ;
**c) A**_{**1**} représente :
1) un halogène,
2) un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R",
3) un cycle hydrocarboné C₆, éventuellement accolé à un cycle C₇ contenant éventuellement au moins un hétéroatome pour former un hétérocycle Hy₆, ces cycles C₆ et C₇ pouvant comporter au moins une fonction carbonyle ou thiocarbonyle et être substitués par au moins un substituant A₅,
4) un hétérocycle Hy₇ éventuellement accolé à un cycle C₇ contenant éventuellement au moins un hétéroatome pour former un hétérocycle Hy₆, ces cycles Hy₇ et C₇ pouvant comporter au moins une fonction carbonyle ou thiocarbonyle et être substitués par au moins un substituant A₅ ;
**d) A**_{**2**} représente :
1) un halogène,
2) un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R", ou
3) un cycle C₈ éventuellement accolé à un cycle C₉, ces cycles C₈ et C₉ contenant éventuellement au moins un hétéroatome pour former un hétérocycle Hy₈ et/ou au moins une fonction carbonyle ou thiocarbonyle et/ou étant substitués par au moins un substituant A₅ ;
**e) A**_{**3**} représente :
1) A₂ , ou
2) un groupe alkyle en C₁-C₂₀ éventuellement substitué par au moins un substituant A₅ ;
**f) A**_{**4**} représente :
1) un halogène,
2) un groupe CF₃, CN, OR, SR, NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R",
3) un groupe alkyle en C₁-C₂₀ éventuellement substitué par au moins un substituant A₅,
4) un cycle C₈ éventuellement accolé à un cycle C₉, ces cycles C₈ et C₉ contenant éventuellement au moins un hétéroatome pour former un hétérocycle Hy₈ et/ou au moins une fonction carbonyle ou thiocarbonyle et/ou étant substitués par au moins un substituant A₅ ;
**g) A**_{**5**} représente :
1) un halogène,
2) un groupe CF₃, CN, OR₅, SR₅, NR₅R₅', NR₅C(=NR₅')NR₅"R₅"', COR₅, CSR₅, COOR₅, CONR₅R₅', CSNR₅R₅', NR₅CSR₅', NR₅CSNR₅'R₅", NR₅COR₅', NR₅CONR₅'R₅", SO₂NR₅R₅', NR₅SO₂R₅', SO₂R₅ ou SiR₅R₅'R₅", R₅, R₅', R₅" étant un atome d'hydrogène ou un groupe alkyle en C₁-C₂₀,
3) un groupe alkyle en C₁-C₂₀, ou
4) un cycle C₁₀ éventuellement accolé à un autre cycle C₁₁, ces cycles contenant éventuellement au moins un hétéroatome pour former un hétérocycle Hy₉ et/ou comportant au moins une fonction carbonyle ou thiocarbonyle ;
**h) R, R', R" et R"'**, identiques ou différents, représentent :
1) un atome d'hydrogène,
2) un groupe alkyle en C₁-C₂₀ éventuellement substitué par au moins un substituant A₅, ou
3) un cycle C₁₂ éventuellement accolé à un autre cycle C₁₃, ces cycles contenant éventuellement au moins un hétéroatome pour former un hétérocycle Hy₁₀ et pouvant être substitués par au moins un substituant A₅ ;
**i) Hy**_{**1**} **à Hy**_{**10**} représentent indépendamment un hétérocycle pouvant contenir de 1 à 4 hétéroatomes choisis parmi N, O et S, dans une composition cosmétique de soin et/ou de maquillage des fibres kératiniques humaines pour induire et/ou stimuler leur croissance, freiner leur chute et/ou augmenter leur densité.

3. Utilisation d'au moins un composé 2-thioacétamide de formule (I) ou d'un de ses sels, 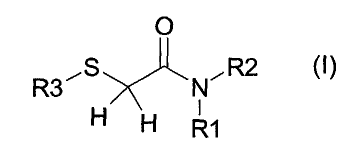 dans laquelle :
**a) R**_{**1**} **et R**_{**2**} représentent indépendamment :
1) un atome d'hydrogène,
2) un radical alkyle en C₁-C₂₀ éventuellement substitué par au moins un substituant A₁,
3) un cycle hydrocarboné C₁, éventuellement accolé à un cycle C₂ contenant éventuellement au moins un hétéroatome pour former un hétérocycle Hy₁, ces cycles C₁ et C₂ pouvant comporter au moins une fonction carbonyle ou thiocarbonyle et être substitués par au moins un substituant A₃,
4) un hétérocycle Hy₂ éventuellement accolé à un cycle C₂ contenant éventuellement au moins un hétéroatome pour former un hétérocycle Hy₃, ces cycles Hy₂ et C₂ pouvant comporter une fonction carbonyle ou thiocarbonyle et être substitués par au moins un substituant A₃, ou
5) un groupe C(=NR)R', C(=NR)NR'R", COR, CSR, COOR, CONRR', SO₂R ou SO₂NRR' ;
**b) R**_{**3**} représente :
1) un radical alkyle en C₁-C₂₀ éventuellement substitué par au moins un substituant A₂,
2) un cycle hydrocarboné C₃, éventuellement accolé à un cycle C₄ contenant éventuellement au moins un hétéroatome pour former un hétérocycle Hy₃, ces cycles C₃ et C₄ pouvant comporter au moins une fonction carbonyle ou thiocarbonyle et être substitués par au moins un substituant A₃,
3) un hétérocycle Hy₄ représentant un cycle pyrrole, furanne, thiophène ou pyrazole, éventuellement accolé à un cycle C₅ représentant un cycle phényle, pyridine ou pyrimidine, ces deux cycles Hy₄ et C₅ pouvant être substitués par au moins un substituant A₃,
4) un cycle pyridine éventuellement accolé à un cycle C₂ contenant éventuellement au moins un hétéroatome pour former un hétérocycle Hy₁, ces cycles pyridine et C₂ pouvant comporter au moins une fonction carbonyle ou thiocarbonyle et être substitués par au moins un substituant A₄,
5) un hétérocycle Hy₅ différent de Hy₄ et du cycle pyridine, éventuellement accolé à un cycle C₂ contenant éventuellement au moins un hétéroatome pour former un hétérocycle Hy₁, ces cycles Hy₅ et C₂ pouvant comporter au moins une fonction carbonyle ou thiocarbonyle et être substitués par au moins un substituant A₃, ou
6) un groupe C(=NR)R', C(=NR)NR'R", COR, CSR, COOR ou CONRR' ;
**c) A**_{**1**} représente :
1) un halogène,
2) un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R",
3) un cycle hydrocarboné C₆, éventuellement accolé à un cycle C₇ contenant éventuellement au moins un hétéroatome pour former un hétérocycle Hy₆, ces cycles C₆ et C₇ pouvant comporter au moins une fonction carbonyle ou thiocarbonyle et être substitués par au moins un substituant A₅,
4) un hétérocycle Hy₇ éventuellement accolé à un cycle C₇ contenant éventuellement au moins un hétéroatome pour former un hétérocycle Hy₆, ces cycles Hy₇ et C₇ pouvant comporter au moins une fonction carbonyle ou thiocarbonyle et être substitués par au moins un substituant A₅ ;
**d) A**_{**2**} représente :
1) un halogène,
2) un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R", ou
3) un cycle C₈ éventuellement accolé à un cycle C₉, ces cycles C₈ et C₉ contenant éventuellement au moins un hétéroatome pour former un hétérocycle Hy₈ et/ou au moins une fonction carbonyle ou thiocarbonyle et/ou étant substitués par au moins un substituant A₅ ;
**e) A**_{**3**} représente :
1) A₂, ou
2) un groupe alkyle en C₁-C₂₀ éventuellement substitué par au moins un substituant A₅ ;
**f) A**_{**4**} représente :
1) un halogène,
2) un groupe CF₃, CN, OR, SR, NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R",
3) un groupe alkyle en C₁-C₂₀ éventuellement substitué par au moins un substituant A₅,
4) un cycle C₈ éventuellement accolé à un cycle C₉, ces cycles C₈ et C₉ contenant éventuellement au moins un hétéroatome pour former un hétérocycle Hy₈ et/ou au moins une fonction carbonyle ou thiocarbonyle et/ou étant substitués par au moins un substituant A₅ ;
**g) A**_{**5**} représente :
1) un halogène,
2) un groupe CF₃, CN, OR₅, SR₅, NR₅R₅', NR₅C(=NR₅')NR₅"R,₅"', COR₅, CSR₅, COOR₅, CONR₅R₅', CSNR₅R₅', NR₅CSR₅', NR₅CSNR₅'R₅", NR₅COR₅', NR₅CONR₅'R₅", SO₂NR₅R₅', NR₅SO₂R₅', SO₂R₅ ou SiR₅R₅'R₅", R₅, R₅', R₅" étant un atome d'hydrogène ou un groupe alkyle en C₁-C₂₀,
3) un groupe alkyle en C₁-C₂₀, ou
4) un cycle C₁₀ éventuellement accolé à un autre cycle C₁₁, ces cycles contenant éventuellement au moins un hétéroatome pour former un hétérocycle Hy₉ et/ou comportant au moins une fonction carbonyle ou thiocarbonyle ;
**h) R, R', R" et R"'**, identiques ou différents, représentent :
1) un atome d'hydrogène, 2)
2) un groupe alkyle en C₁-C₂₀ éventuellement substitué par au moins un substituant A₅, ou
3) un cycle C₁₂ éventuellement accolé à un autre cycle C₁₃, ces cycles contenant éventuellement au moins un hétéroatome pour former un hétérocycle Hy₁₀ et pouvant être substitués par au moins un substituant A₅ ;
**i) Hy**_{**1**} **à Hy**_{**10**} représentent indépendamment un hétérocycle pouvant contenir de 1 à 4 hétéroatomes choisis parmi N, O et S, pour la fabrication d'une composition de soin ou de traitement des fibres kératiniques humaines, destinée à induire et/ou stimuler la croissance des fibres et/ou freiner leur chute et/ou augmenter leur densité.

4. Utilisation d'au moins un composé 2-thioacétamide de formule (I) ou de l'un de ses sels, 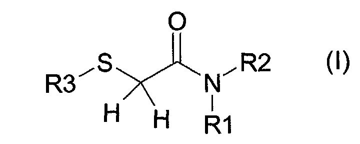 dans laquelle :
**a) R**_{**1**} **et R**_{**2**} représentent indépendamment :
1) un atome d'hydrogène,
2) un radical alkyle en C₁-C₂₀ éventuellement substitué par au moins un substituant A₁,
3) un cycle hydrocarboné C₁, éventuellement accolé à un cycle C₂ contenant éventuellement au moins un hétéroatome pour former un hétérocycle Hy₁, ces cycles C₁ et C₂ pouvant comporter au moins une fonction carbonyle ou thiocarbonyle et être substitués par au moins un substituant A₃,
4) un hétérocycle Hy₂ éventuellement accolé à un cycle C₂ contenant éventuellement au moins un hétéroatome pour former un hétérocycle Hy₃, ces cycles Hy₂ et C₂ pouvant comporter une fonction carbonyle ou thiocarbonyle et être substitués par au moins un substituant A₃, ou
5) un groupe C(=NR)R', C(=NR)NR'R", COR, CSR, COOR, CONRR', SO₂R ou SO₂NRR' ;
**b) R**_{**3**} représente :
1) un radical alkyle en C₁-C₂₀ éventuellement substitué par au moins un substituant A₂,
2) un cycle hydrocarboné C₃, éventuellement accolé à un cycle C₄ contenant éventuellement au moins un hétéroatome pour former un hétérocycle Hy₃, ces cycles C₃ et C₄ pouvant comporter au moins une fonction carbonyle ou thiocarbonyle et être substitués par au moins un substituant A₃,
3) un hétérocycle Hy₄ représentant un cycle pyrrole, furanne, thiophène ou pyrazole, éventuellement accolé à un cycle C₅ représentant un cycle phényle, pyridine ou pyrimidine, ces deux cycles Hy₄ et C₅ pouvant être substitués par au moins un substituant A₃,
4) un cycle pyridine éventuellement accolé à un cycle C₂ contenant éventuellement au moins un hétéroatome pour former un hétérocycle Hy₁, ces cycles pyridine et C₂ pouvant comporter au moins une fonction carbonyle ou thiocarbonyle et être substitués par au moins un substituant A₄,
5) un hétérocycle Hy₅ différent de Hy₄ et du cycle pyridine, éventuellement accolé à un cycle C₂ contenant éventuellement au moins un hétéroatome pour former un hétérocycle Hy₁, ces cycles Hy₅ et C₂ pouvant comporter au moins une fonction carbonyle ou thiocarbonyle et être substitués par au moins un substituant A₃, ou
6) un groupe C(=NR)R', C(=NR)NR'R", COR, CSR, COOR ou CONRR' ;
**c) A**_{**1**} représente :
1) un halogène,
2) un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R",
3) un cycle hydrocarboné C₆, éventuellement accolé à un cycle C₇ contenant éventuellement au moins un hétéroatome pour former un hétérocycle Hy₆, ces cycles C₆ et C₇ pouvant comporter au moins une fonction carbonyle ou thiocarbonyle et être substitués par au moins un substituant A₅,
4) un hétérocycle Hy₇ éventuellement accolé à un cycle C₇ contenant éventuellement au moins un hétéroatome pour former un hétérocycle Hy₆, ces cycles Hy₇ et C₇ pouvant comporter au moins une fonction carbonyle ou thiocarbonyle et être substitués par au moins un substituant A₅ ;
**d) A**_{**2**} représente :
1) un halogène,
2) un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R", ou
3) un cycle C₈ éventuellement accolé à un cycle C₉, ces cycles C₈ et C₉ contenant éventuellement au moins un hétéroatome pour former un hétérocycle Hy₈ et/ou au moins une fonction carbonyle ou thiocarbonyle et/ou étant substitués par au moins un substituant A₅ ;
**e) A**_{**3**} représente :
1) A₂ , ou
2) un groupe alkyle en C₁-C₂₀ éventuellement substitué par au moins un substituant A₅ ;
**f) A**_{**4**} représente :
1) un halogène,
2) un groupe CF₃, CN, OR, SR, NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R",
3) un groupe alkyle en C₁-C₂₀ éventuellement substitué par au moins un substituant A₅,
4) un cycle C₈ éventuellement accolé à un cycle C₉, ces cycles C₈ et C₉ contenant éventuellement au moins un hétéroatome pour former un hétérocycle Hy₈ et/ou au moins une fonction carbonyle ou thiocarbonyle et/ou étant substitués par au moins un substituant A₅ ;
**g) A**_{**5**} représente :
1) un halogène,
2) un groupe CF₃, CN, OR₅, SR₅, NR₅R₅', NR₅C(=NR₅')NR₅"R₅"', COR₅, CSR₅, COOR₅, CONR₅R₅', CSNR₅R₅', NR₅CSR₅', NR₅CSNR₅'R₅", NR₅COR₅', NR₅CONR₅'R₅", SO₂NR₅R₅', NR₅SO₂R₅', SO₂R₅ ou SiR₅R₅'R₅", R₅, R₅', R₅" étant un atome d'hydrogène ou un groupe alkyle en C₁-C₂₀,
3) un groupe alkyle en C₁-C₂₀, ou
4) un cycle C₁₀ éventuellement accolé à un autre cycle C₁₁, ces cycles contenant éventuellement au moins un hétéroatome pour former un hétérocycle Hy₉ et/ou comportant au moins une fonction carbonyle ou thiocarbonyle ;
**h) R, R', R" et R"'**, identiques ou différents, représentent :
1) un atome d'hydrogène,
2) un groupe alkyle en C₁-C₂₀ éventuellement substitué par au moins un substituant A₅, ou
3) un cycle C₁₂ éventuellement accolé à un autre cycle C₁₃, ces cycles contenant éventuellement au moins un hétéroatome pour former un hétérocycle Hy₁₀ et pouvant être substitués par au moins un substituant A₅.
**i) Hy**_{**1**} **à Hy**_{**10**} représentent indépendamment un hétérocycle pouvant contenir de 1 à 4 hétéroatomes choisis parmi N, O et S,
comme agent inhibiteur de la 15-hydroxy prostaglandine déshydrogénase.

5. Utilisation d'au moins un composé 2-thioacétamide de formule (I) ou de l'un de ses sels, 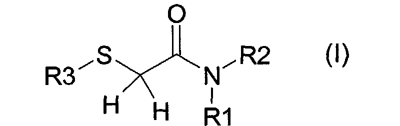 dans laquelle :
**a) R**_{**1**} **et R**_{**2**} représentent indépendamment :
1) un atome d'hydrogène,
2) un radical alkyle en C₁-C₂₀ éventuellement substitué par au moins un substituant A₁,
3) un cycle hydrocarboné C₁, éventuellement accolé à un cycle C₂ contenant éventuellement au moins un hétéroatome pour former un hétérocycle Hy₁, ces cycles C₁ et C₂ pouvant comporter au moins une fonction carbonyle ou thiocarbonyle et être substitués par au moins un substituant A₃,
4) un hétérocycle Hy₂ éventuellement accolé à un cycle C₂ contenant éventuellement au moins un hétéroatome pour former un hétérocycle Hy₃, ces cycles Hy₂ et C₂ pouvant comporter une fonction carbonyle ou thiocarbonyle et être substitués par au moins un substituant A₃, ou
5) un groupe C(=NR)R', C(=NR)NR'R", COR, CSR, COOR, CONRR', SO₂R ou SO₂NRR' ;
**b) R**_{**3**} représente :
1) un radical alkyle en C₁-C₂₀ éventuellement substitué par au moins un substituant A₂,
2) un cycle hydrocarboné C₃, éventuellement accolé à un cycle C₄ contenant éventuellement au moins un hétéroatome pour former un hétérocycle Hy₃, ces cycles C₃ et C₄ pouvant comporter au moins une fonction carbonyle ou thiocarbonyle et être substitués par au moins un substituant A₃,
3) un hétérocycle Hy₄ représentant un cycle pyrrole, furanne, thiophène ou pyrazole, éventuellement accolé à un cycle C₅ représentant un cycle phényle, pyridine ou pyrimidine, ces deux cycles Hy₄ et C₅ pouvant être substitués par au moins un substituant A₃,
4) un cycle pyridine éventuellement accolé à un cycle C₂ contenant éventuellement au moins un hétéroatome pour former un hétérocycle Hy₁, ces cycles pyridine et C₂ pouvant comporter au moins une fonction carbonyle ou thiocarbonyle et être substitués par au moins un substituant A₄,
5) un hétérocycle Hy₅ différent de Hy₄ et du cycle pyridine, éventuellement accolé à un cycle C₂ contenant éventuellement au moins un hétéroatome pour former un hétérocycle Hy₁, ces cycles Hy₅ et C₂ pouvant comporter au moins une fonction carbonyle ou thiocarbonyle et être substitués par au moins un substituant A₃, ou
6) un groupe C(=NR)R', C(=NR)NR'R", COR, CSR, COOR ou CONRR' ;
**c) A**_{**1**} représente :
1) un halogène,
2) un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R",
3) un cycle hydrocarboné C₆, éventuellement accolé à un cycle C₇ contenant éventuellement au moins un hétéroatome pour former un hétérocycle Hy₆, ces cycles C₆ et C₇ pouvant comporter au moins une fonction carbonyle ou thiocarbonyle et être substitués par au moins un substituant A₅,
4) un hétérocycle Hy₇ éventuellement accolé à un cycle C₇ contenant éventuellement au moins un hétéroatome pour former un hétérocycle Hy₆, ces cycles Hy₇ et C₇ pouvant comporter au moins une fonction carbonyle ou thiocarbonyle et être substitués par au moins un substituant A₅ ;
**d) A**_{**2**} représente :
1) un halogène,
2) un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R", ou
3) un cycle C₈ éventuellement accolé à un cycle C₉, ces cycles C₈ et C₉ contenant éventuellement au moins un hétéroatome pour former un hétérocycle Hy₈ et/ou au moins une fonction carbonyle ou thiocarbonyle et/ou étant substitués par au moins un substituant A₅ ;
**e) A**_{**3**} représente :
1) A₂, ou
2) un groupe alkyle en C₁-C₂₀ éventuellement substitué par au moins un substituant A₅ ;
**f) A**_{**4**} représente :
1) un halogène,
2) un groupe CF₃, CN, OR, SR, NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R",
3) un groupe alkyle en C₁-C₂₀ éventuellement substitué par au moins un substituant A₅,
4) un cycle C₈ éventuellement accolé à un cycle C₉, ces cycles C₈ et C₉ contenant éventuellement au moins un hétéroatome pour former un hétérocycle Hy₈ et/ou au moins une fonction carbonyle ou thiocarbonyle et/ou étant substitués par au moins un substituant A₅ ;
**g) A**_{**5**} représente :
1) un halogène,
2) un groupe CF₃, CN, OR₅, SR₅, NR₅R₅', NR₅C(=NR₅')NR₅"R₅"', COR₅, CSR₅, COOR₅, CONR₅R₅', CSNR₅R₅', NR₅CSR₅', NR₅CSNR₅'R₅", NR₅COR₅', NR₅CONR₅'R₅", SO₂NR₅R₅', NR₅SO₂R₅', SO₂R₅ ou SiR₅R₅'R₅", R₅, R₅', R₅" étant un atome d'hydrogène ou un groupe alkyle en C₁-C₂₀,
3) un groupe alkyle en C₁-C₂₀, ou
4) un cycle C₁₀ éventuellement accolé à un autre cycle C₁₁, ces cycles contenant éventuellement au moins un hétéroatome pour former un hétérocycle Hy₉ et/ou comportant au moins une fonction carbonyle ou thiocarbonyle ;
**h) R, R', R" et R"'**, identiques ou différents, représentent :
1) un atome d'hydrogène,
2) un groupe alkyle en C₁-C₂₀ éventuellement substitué par au moins un substituant A₅, ou
3) un cycle C₁₂ éventuellement accolé à un autre cycle C₁₃, ces cycles contenant éventuellement au moins un hétéroatome pour former un hétérocycle Hy₁₀ et pouvant être substitués par au moins un substituant A₅ ;
**i) Hy**_{**1**} **à Hy**_{**10**} représentent indépendamment un hétérocycle pouvant contenir de 1 à 4 hétéroatomes choisis parmi N, O et S,
pour la fabrication d'une composition de soin ou de traitement des fibres kératiniques humaines, destinée à traiter les désordres liés à la 15-hydroxy prostaglandine déshydrogénase.

6. Utilisation selon l'une des revendications précédentes, **caractérisée en ce que** les fibres kératiniques sont les cheveux, les sourcils, les cils, les poils de barbe, de moustache et les poils pubiens.

7. Utilisation cosmétique d'au moins un composé 2-thioacétamide de formule (I) ou d'un de ses sels, 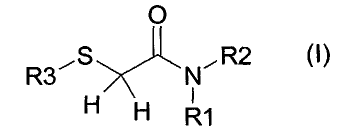 dans laquelle :
**a) R**_{**1**} **et R**_{**2**} représentent indépendamment :
1) un atome d'hydrogène,
2) un radical alkyle en C₁-C₂₀ éventuellement substitué par au moins un substituant A₁,
3) un cycle hydrocarboné C₁, éventuellement accolé à un cycle C₂ contenant éventuellement au moins un hétéroatome pour former un hétérocycle Hy₁, ces cycles C₁ et C₂ pouvant comporter au moins une fonction carbonyle ou thiocarbonyle et être substitués par au moins un substituant A₃,
4) un hétérocycle Hy₂ éventuellement accolé à un cycle C₂ contenant éventuellement au moins un hétéroatome pour former un hétérocycle Hy₃, ces cycles Hy₂ et C₂ pouvant comporter une fonction carbonyle ou thiocarbonyle et être substitués par au moins un substituant A₃, ou
5) un groupe C(=NR)R', C(=NR)NR'R", COR, CSR, COOR, CONRR', SO₂R ou SO₂NRR' ;
**b) R**_{**3**} représente :
1) un radical alkyle en C₁-C₂₀ éventuellement substitué par au moins un substituant A₂,
2) un cycle hydrocarboné C₃, éventuellement accolé à un cycle C₄ contenant éventuellement au moins un hétéroatome pour former un hétérocycle Hy₃, ces cycles C₃ et C₄ pouvant comporter au moins une fonction carbonyle ou thiocarbonyle et être substitués par au moins un substituant A₃,
3) un hétérocycle Hy₄ représentant un cycle pyrrole, furanne, thiophène ou pyrazole, éventuellement accolé à un cycle C₅ représentant un cycle phényle, pyridine ou pyrimidine, ces deux cycles Hy₄ et C₅ pouvant être substitués par au moins un substituant A₃,
4) un cycle pyridine éventuellement accolé à un cycle C₂ contenant éventuellement au moins un hétéroatome pour former un hétérocycle Hy₁, ces cycles pyridine et C₂ pouvant comporter au moins une fonction carbonyle ou thiocarbonyle et être substitués par au moins un substituant A₄,
5) un hétérocycle Hy₅ différent de Hy₄ et du cycle pyridine, éventuellement accolé à un cycle C₂ contenant éventuellement au moins un hétéroatome pour former un hétérocycle Hy₁, ces cycles Hy₅ et C₂ pouvant comporter au moins une fonction carbonyle ou thiocarbonyle et être substitués par au moins un substituant A₃, ou
6) un groupe C(=NR)R', C(=NR)NR'R", COR, CSR, COOR ou CONRR' ;
**c) A**_{**1**} représente :
1) un halogène,
2) un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R",
3) un cycle hydrocarboné C₆, éventuellement accolé à un cycle C₇ contenant éventuellement au moins un hétéroatome pour former un hétérocycle Hy₆, ces cycles C₆ et C₇ pouvant comporter au moins une fonction carbonyle ou thiocarbonyle et être substitués par au moins un substituant A₅,
4) un hétérocycle Hy₇ éventuellement accolé à un cycle C₇ contenant éventuellement au moins un hétéroatome pour former un hétérocycle Hy₆, ces cycles Hy₇ et C₇ pouvant comporter au moins une fonction carbonyle ou thiocarbonyle et être substitués par au moins un substituant A₅ ;
**d) A**_{**2**} représente :
1) un halogène,
2) un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R", ou
3) un cycle C₈ éventuellement accolé à un cycle C₉, ces cycles C₈ et C₉ contenant éventuellement au moins un hétéroatome pour former un hétérocycle Hy₈ et/ou au moins une fonction carbonyle ou thiocarbonyle et/ou étant substitués par au moins un substituant A₅ ;
**e) A**_{**3**} représente :
1) A₂ , ou
2) un groupe alkyle en C₁-C₂₀ éventuellement substitué par au moins un substituant A₅ ;
**f) A**_{**4**} représente :
1) un halogène,
2) un groupe CF₃, CN, OR, SR, NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R",
3) un groupe alkyle en C₁-C₂₀ éventuellement substitué par au moins un substituant A₅,
4) un cycle C₈ éventuellement accolé à un cycle C₉, ces cycles C₈ et C₉ contenant éventuellement au moins un hétéroatome pour former un hétérocycle Hy₈ et/ou au moins une fonction carbonyle ou thiocarbonyle et/ou étant substitués par au moins un substituant A₅ ;
**g) A**_{**5**} représente :
1) un halogène,
2) un groupe CF₃, CN, OR₅, SR₅, NR₅R₅', NR₅C(=NR₅')NR₅"R₅"', COR₅, CSR₅, COOR₅, CONR₅R₅', CSNR₅R₅', NR₅CSR₅', NR₅CSNR₅'R₅", NR₅COR₅', NR₅CONR₅'R₅", SO₂NR₅R₅', NR₅SO₂R₅', SO₂R₅ ou SiR₅R₅'R₅", R₅, R₅', R₅" étant un atome d'hydrogène ou un groupe alkyle en C₁-C₂₀,
3) un groupe alkyle en C₁-C₂₀, ou
4) un cycle C₁₀ éventuellement accolé à un autre cycle C₁₁, ces cycles contenant éventuellement au moins un hétéroatome pour former un hétérocycle Hy₉ et/ou comportant au moins une fonction carbonyle ou thiocarbonyle ;
**h) R, R', R" et R"'**, identiques ou différents, représentent :
1) un atome d'hydrogène,
2) un groupe alkyle en C₁-C₂₀ éventuellement substitué par au moins un substituant A₅, ou
3) un cycle C₁₂ éventuellement accolé à un autre cycle C₁₃, ces cycles contenant éventuellement au moins un hétéroatome pour former un hétérocycle Hy₁₀ et pouvant être substitués par au moins un substituant A₅ ;
**i) Hy**_{**1**} **à Hy**_{**10**} représentent indépendamment un hétérocycle pouvant contenir de 1 à 4 hétéroatomes choisis parmi N, O et S,
dans une composition cosmétique de soin capillaire d'être humain pour réduire la chute des cheveux et/ou augmenter leur densité et/ou traiter l'alopécie andro-chrono-génétique et/ou traiter l'alopécie d'origine naturelle.

8. Utilisation d'au moins un composé 2-thioacétamide de formule (I) ou d'un de ses sels. 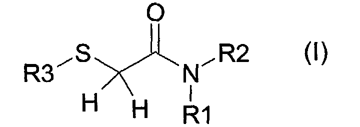 dans laquelle :
**a) R**_{**1**} **et R**_{**2**} représentent indépendamment :
1) un atome d'hydrogène,
2) un radical alkyle en C₁-C₂₀ éventuellement substitué par au moins un substituant A₁,
3) un cycle hydrocarboné C₁, éventuellement accolé à un cycle C₂ contenant éventuellement au moins un hétéroatome pour former un hétérocycle Hy₁, ces cycles C₁ et C₂ pouvant comporter au moins une fonction carbonyle ou thiocarbonyle et être substitués par au moins un substituant A₃,
4) un hétérocycle Hy₂ éventuellement accolé à un cycle C₂ contenant éventuellement au moins un hétéroatome pour former un hétérocycle Hy₃, ces cycles Hy₂ et C₂ pouvant comporter une fonction carbonyle ou thiocarbonyle et être substitués par au moins un substituant A₃, ou
5) un groupe C(=NR)R', C(=NR)NR'R", COR, CSR, COOR, CONRR', SO₂R ou SO₂NRR' ;
**b) R**_{**3**} représente :
1) un radical alkyle en C₁-C₂₀ éventuellement substitué par au moins un substituant A₂,
2) un cycle hydrocarboné C₃, éventuellement accolé à un cycle C₄ contenant éventuellement au moins un hétéroatome pour former un hétérocycle Hy₃, ces cycles C₃ et C₄ pouvant comporter au moins une fonction carbonyle ou thiocarbonyle et être substitués par au moins un substituant A₃,
3) un hétérocycle Hy₄ représentant un cycle pyrrole, furanne, thiophène ou pyrazole, éventuellement accolé à un cycle C₅ représentant un cycle phényle, pyridine ou pyrimidine, ces deux cycles Hy₄ et C₅ pouvant être substitués par au moins un substituant A₃,
4) un cycle pyridine éventuellement accolé à un cycle C₂ contenant éventuellement au moins un hétéroatome pour former un hétérocycle Hy₁, ces cycles pyridine et C₂ pouvant comporter au moins une fonction carbonyle ou thiocarbonyle et être substitués par au moins un substituant A₄,
5) un hétérocycle Hy₅ différent de Hy₄ et du cycle pyridine, éventuellement accolé à un cycle C₂ contenant éventuellement au moins un hétéroatome pour former un hétérocycle Hy₁, ces cycles Hy₅ et C₂ pouvant comporter au moins une fonction carbonyle ou thiocarbonyle et être substitués par au moins un substituant A₃, ou
6) un groupe C(=NR)R', C(=NR)NR'R", COR, CSR, COOR ou CONRR' ;
**c) A**_{**1**} représente :
1) un halogène,
2) un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R",
3) un cycle hydrocarboné C₆, éventuellement accolé à un cycle C₇ contenant éventuellement au moins un hétéroatome pour former un hétérocycle Hy₆, ces cycles C₆ et C₇ pouvant comporter au moins une fonction carbonyle ou thiocarbonyle et être substitués par au moins un substituant A₅,
4) un hétérocycle Hy₇ éventuellement accolé à un cycle C₇ contenant éventuellement au moins un hétéroatome pour former un hétérocycle Hy₆, ces cycles Hy₇ et C₇ pouvant comporter au moins une fonction carbonyle ou thiocarbonyle et être substitués par au moins un substituant A₅ ;
**d) A**_{**2**} représente :
1) un halogène,
2) un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R", ou
3) un cycle C₈ éventuellement accolé à un cycle C₉, ces cycles C₈ et C₉ contenant éventuellement au moins un hétéroatome pour former un hétérocycle Hy₈ et/ou au moins une fonction carbonyle ou thiocarbonyle et/ou étant substitués par au moins un substituant A₅ ;
**e) A**_{**3**} représente :
1) A₂ , ou
2) un groupe alkyle en C₁-C₂₀ éventuellement substitué par au moins un substituant A₅ ;
**f) A**_{**4**} représente :
1) un halogène,
2) un groupe CF₃, CN, OR, SR, NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R",
3) un groupe alkyle en C₁-C₂₀ éventuellement substitué par au moins un substituant A₅,
4) un cycle C₈ éventuellement accolé à un cycle C₉, ces cycles C₈ et C₉ contenant éventuellement au moins un hétéroatome pour former un hétérocycle Hy₈ et/ou au moins une fonction carbonyle ou thiocarbonyle et/ou étant substitués par au moins un substituant A₅ ;
**g) A**_{**5**} représente :
1) un halogène,
2) un groupe CF₃, CN, OR₅, SR₅, NR₅R₅', NR₅C(=NR₅')NR₅"R₅"', COR₅, CSR₅, COOR₅, CONR₅R₅', CSNR₅R₅', NR₅CSR₅', NR₅CSNR₅'R₅", NR₅COR₅', NR₅CONR₅'R₅", SO₂NR₅R₅', NR₅SO₂R₅', SO₂R₅ ou SiR₅R₅'R₅", R₅, R₅', R₅" étant un atome d'hydrogène ou un groupe alkyle en C₁-C₂₀,
3) un groupe alkyle en C₁-C₂₀, ou
4) un cycle C₁₀ éventuellement accolé à un autre cycle C₁₁, ces cycles contenant éventuellement au moins un hétéroatome pour former un hétérocycle Hy₉ et/ou comportant au moins une fonction carbonyle ou thiocarbonyle ;
**h) R, R', R" et R"'**, identiques ou différents, représentent :
1) un atome d'hydrogène,
2) un groupe alkyle en C₁-C₂₀ éventuellement substitué par au moins un substituant A₅, ou
3) un cycle C₁₂ éventuellement accolé à un autre cycle C₁₃, ces cycles contenant éventuellement au moins un hétéroatome pour former un hétérocycle Hy₁₀ et pouvant être substitués par au moins un substituant A₅ ;
**i) Hy**_{**1**} **à Hy**_{**10**} représentent indépendamment un hétérocycle pouvant contenir de 1 à 4 hétéroatomes choisis parmi N, O et S,
pour la préparation d'une composition capillaire d'être humain, destinée à induire et/ou stimuler la croissance des cheveux et/ou freiner leur chute et/ou augmenter leur densité et/ou traiter l'alopécie androgénique et/ou traiter l'alopécie d'origine naturelle.

9. Utilisation cosmétique d'au moins un composé 2-thioacétamide de formule (I) ou de l'un de ses sels 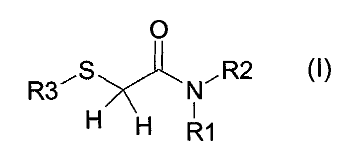 dans laquelle :
**a) R**_{**1**} **et R**_{**2**} représentent indépendamment :
1) un atome d'hydrogène,
2) un radical alkyle en C₁-C₂₀ éventuellement substitué par au moins un substituant A₁,
3) un cycle hydrocarboné C₁, éventuellement accolé à un cycle C₂ contenant éventuellement au moins un hétéroatome pour former un hétérocycle Hy₁, ces cycles C₁ et C₂ pouvant comporter au moins une fonction carbonyle ou thiocarbonyle et être substitués par au moins un substituant A₃,
4) un hétérocycle Hy₂ éventuellement accolé à un cycle C₂ contenant éventuellement au moins un hétéroatome pour former un hétérocycle Hy₃, ces cycles Hy₂ et C₂ pouvant comporter une fonction carbonyle ou thiocarbonyle et être substitués par au moins un substituant A₃, ou
5) un groupe C(=NR)R', C(=NR)NR'R", COR, CSR, COOR, CONRR', SO₂R ou SO₂NRR' ;
**b) R**_{**3**} représente :
1) un radical alkyle en C₁-C₂₀ éventuellement substitué par au moins un substituant A₂,
2) un cycle hydrocarboné C₃, éventuellement accolé à un cycle C₄ contenant éventuellement au moins un hétéroatome pour former un hétérocycle Hy₃, ces cycles C₃ et C₄ pouvant comporter au moins une fonction carbonyle ou thiocarbonyle et être substitués par au moins un substituant A₃,
3) un hétérocycle Hy₄ représentant un cycle pyrrole, furanne, thiophène ou pyrazole, éventuellement accolé à un cycle C₅ représentant un cycle phényle, pyridine ou pyrimidine, ces deux cycles Hy₄ et C₅ pouvant être substitués par au moins un substituant A₃,
4) un cycle pyridine éventuellement accolé à un cycle C₂ contenant éventuellement au moins un hétéroatome pour former un hétérocycle Hy₁, ces cycles pyridine et C₂ pouvant comporter au moins une fonction carbonyle ou thiocarbonyle et être substitués par au moins un substituant A₄,
5) un hétérocycle Hy₅ différent de Hy₄ et du cycle pyridine, éventuellement accolé à un cycle C₂ contenant éventuellement au moins un hétéroatome pour former un hétérocycle Hy₁, ces cycles Hy₅ et C₂ pouvant comporter au moins une fonction carbonyle ou thiocarbonyle et être substitués par au moins un substituant A₃, ou
6) un groupe C(=NR)R', C(=NR)NR'R", COR, CSR, COOR ou CONRR' ;
**c) A**_{**1**} représente :
1) un halogène,
2) un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R",
3) un cycle hydrocarboné C₆, éventuellement accolé à un cycle C₇ contenant éventuellement au moins un hétéroatome pour former un hétérocycle Hy₆, ces cycles C₆ et C₇ pouvant comporter au moins une fonction carbonyle ou thiocarbonyle et être substitués par au moins un substituant A₅,
4) un hétérocycle Hy₇ éventuellement accolé à un cycle C₇ contenant éventuellement au moins un hétéroatome pour former un hétérocycle Hy₆, ces cycles Hy₇ et C₇ pouvant comporter au moins une fonction carbonyle ou thiocarbonyle et être substitués par au moins un substituant A₅ ;
**d) A**_{**2**} représente :
1) un halogène,
2) un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R", ou
3) un cycle C₈ éventuellement accolé à un cycle C₉, ces cycles C₈ et C₉ contenant éventuellement au moins un hétéroatome pour former un hétérocycle Hy₈ et/ou au moins une fonction carbonyle ou thiocarbonyle et/ou étant substitués par au moins un substituant A₅ ;
**e) A**_{**3**} représente :
1) A₂, ou
2) un groupe alkyle en C₁-C₂₀ éventuellement substitué par au moins un substituant A₅ ;
**f) A**_{**4**} représente :
1) un halogène,
2) un groupe CF₃, CN, OR, SR, NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R",
3) un groupe alkyle en C₁-C₂₀ éventuellement substitué par au moins un substituant A₅,
4) un cycle C₈ éventuellement accolé à un cycle C₉, ces cycles C₈ et C₉ contenant éventuellement au moins un hétéroatome pour former un hétérocycle Hy₈ et/ou au moins une fonction carbonyle ou thiocarbonyle et/ou étant substitués par au moins un substituant A₅ ;
**g) A**_{**5**} représente :
1) un halogène,
2) un groupe CF₃, CN, OR₅, SR₅, NR₅R₅', NR₅C(=NR₅')NR₅"R₅"', COR₅, CSR₅, COOR₅, CONR₅R₅', CSNR₅R₅', NR₅CSR₅', NR₅CSNR₅'R₅", NR₅COR₅', NR₅CONR₅'R₅", SO₂NR₅R₅', NR₅SO₂R₅', SO₂R₅ ou SiR₅R₅'R₅", R₅, R₅', R₅" étant un atome d'hydrogène ou un groupe alkyle en C₁-C₂₀,
3) un groupe alkyle en C₁-C₂₀, ou
4) un cycle C₁₀ éventuellement accolé à un autre cycle C₁₁, ces cycles contenant éventuellement au moins un hétéroatome pour former un hétérocycle Hy₉ et/ou comportant au moins une fonction carbonyle ou thiocarbonyle ;
**h) R, R', R" et R"'**, identiques ou différents, représentent :
1) un atome d'hydrogène,
2) un groupe alkyle en C₁-C₂₀ éventuellement substitué par au moins un substituant A₅, ou
3) 3) un cycle C₁₂ éventuellement accolé à un autre cycle C₁₃, ces cycles contenant éventuellement au moins un hétéroatome pour former un hétérocycle Hy₁₀ et pouvant être substitués par au moins un substituant A₅ ;
**i) Hy**_{**1**} **à Hy**_{**10**} représentent indépendamment un hétérocycle pouvant contenir de 1 à 4 hétéroatomes choisis parmi N, O et S,
dans une composition cosmétique de soin et/ou de maquillage des cils humains pour induire et/ou stimuler la croissance des cils et/ou augmenter leur densité.

10. Utilisation d'au moins un composé 2-thioacétamide de formule (I) ou d'un de ses sels 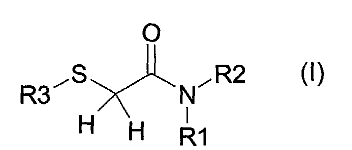 dans laquelle :
**a) R**_{**1**} **et R**_{**2**} représentent indépendamment :
1) un atome d'hydrogène,
2) un radical alkyle en C₁-C₂₀ éventuellement substitué par au moins un substituant A₁,
3) un cycle hydrocarboné C₁, éventuellement accolé à un cycle C₂ contenant éventuellement au moins un hétéroatome pour former un hétérocycle Hy₁, ces cycles C₁ et C₂ pouvant comporter au moins une fonction carbonyle ou thiocarbonyle et être substitués par au moins un substituant A₃,
4) un hétérocycle Hy₂ éventuellement accolé à un cycle C₂ contenant éventuellement au moins un hétéroatome pour former un hétérocycle Hy₃, ces cycles Hy₂ et C₂ pouvant comporter une fonction carbonyle ou thiocarbonyle et être substitués par au moins un substituant A₃, ou
5) un groupe C(=NR)R', C(=NR)NR'R", COR, CSR, COOR, CONRR', SO₂R ou SO₂NRR' ;
**b) R**_{**3**} représente :
1) un radical alkyle en C₁-C₂₀ éventuellement substitué par au moins un substituant A₂,
2) un cycle hydrocarboné C₃, éventuellement accolé à un cycle C₄ contenant éventuellement au moins un hétéroatome pour former un hétérocycle Hy₃, ces cycles C₃ et C₄ pouvant comporter au moins une fonction carbonyle ou thiocarbonyle et être substitués par au moins un substituant A₃,
3) un hétérocycle Hy₄ représentant un cycle pyrrole, furanne, thiophène ou pyrazole, éventuellement accolé à un cycle C₅ représentant un cycle phényle, pyridine ou pyrimidine, ces deux cycles Hy₄ et C₅ pouvant être substitués par au moins un substituant A₃,
4) un cycle pyridine éventuellement accolé à un cycle C₂ contenant éventuellement au moins un hétéroatome pour former un hétérocycle Hy₁, ces cycles pyridine et C₂ pouvant comporter au moins une fonction carbonyle ou thiocarbonyle et être substitués par au moins un substituant A₄,
5) un hétérocycle Hy₅ différent de Hy₄ et du cycle pyridine, éventuellement accolé à un cycle C₂ contenant éventuellement au moins un hétéroatome pour former un hétérocycle Hy₁, ces cycles Hy₅ et C₂ pouvant comporter au moins une fonction carbonyle ou thiocarbonyle et être substitués par au moins un substituant A₃, ou
6) un groupe C(=NR)R', C(=NR)NR'R", COR, CSR, COOR ou CONRR' ;
**c) A**_{**1**} représente :
1) un halogène,
2) un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R",
3) un cycle hydrocarboné C₆, éventuellement accolé à un cycle C₇ contenant éventuellement au moins un hétéroatome pour former un hétérocycle Hy₆, ces cycles C₆ et C₇ pouvant comporter au moins une fonction carbonyle ou thiocarbonyle et être substitués par au moins un substituant A₅,
4) un hétérocycle Hy₇ éventuellement accolé à un cycle C₇ contenant éventuellement au moins un hétéroatome pour former un hétérocycle Hy₆, ces cycles Hy₇ et C₇ pouvant comporter au moins une fonction carbonyle ou thiocarbonyle et être substitués par au moins un substituant A₅ ;
**d) A**_{**2**} représente :
1) un halogène,
2) un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R", ou
3) un cycle C₈ éventuellement accolé à un cycle C₉, ces cycles C₈ et C₉ contenant éventuellement au moins un hétéroatome pour former un hétérocycle Hy₈ et/ou au moins une fonction carbonyle ou thiocarbonyle et/ou étant substitués par au moins un substituant A₅ ;
**e) A**_{**3**} représente :
1) A₂ , ou
2) un groupe alkyle en C₁-C₂₀ éventuellement substitué par au moins un substituant A₅ ;
**f) A**_{**4**} représente :
1) un halogène,
2) un groupe CF₃, CN, OR, SR, NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R",
3) un groupe alkyle en C₁-C₂₀ éventuellement substitué par au moins un substituant A₅,
4) un cycle C₈ éventuellement accolé à un cycle C₉, ces cycles C₈ et C₉ contenant éventuellement au moins un hétéroatome pour former un hétérocycle Hy₈ et/ou au moins une fonction carbonyle ou thiocarbonyle et/ou étant substitués par au moins un substituant A₅ ;
**g) A**_{**5**} représente :
1) un halogène,
2) un groupe CF₃, CN, OR₅, SR₅, NR₅R₅', NR₅C(=NR₅')NR₅"R₅"', COR₅, CSR₅, COOR₅, CONR₅R₅', CSNR₅R₅', NR₅CSR₅', NR₅CSNR₅'R₅", NR₅COR₅', NR₅CONR₅'R₅", SO₂NR₅R₅', NR₅SO₂R₅', SO₂R₅ ou SiR₅R₅'R₅", R₅, R₅', R₅" étant un atome d'hydrogène ou un groupe alkyle en C₁-C₂₀,
3) un groupe alkyle en C₁-C₂₀, ou
4) un cycle C₁₀ éventuellement accolé à un autre cycle C₁₁, ces cycles contenant éventuellement au moins un hétéroatome pour former un hétérocycle Hy₉ et/ou comportant au moins une fonction carbonyle ou thiocarbonyle ;
**h) R, R', R" et R"'**, identiques ou différents, représentent :
1) un atome d'hydrogène,
2) un groupe alkyle en C₁-C₂₀ éventuellement substitué par au moins un substituant A₅, ou
3) un cycle C₁₂ éventuellement accolé à un autre cycle C₁₃, ces cycles contenant éventuellement au moins un hétéroatome pour former un hétérocycle Hy₁₀ et pouvant être substitués par au moins un substituant A₅ ;
i) **Hy**_{**1**} **à Hy**_{**10**} représentent indépendamment un hétérocycle pouvant contenir de 1 à 4 hétéroatomes choisis parmi N, O et S,
pour la préparation d'une composition de soin et/ou de traitement des cils humains destinée à induire et/ou stimuler la croissance des cils et/ou augmenter leur densité.

11. Utilisation selon l'une des revendications précédentes, **caractérisée en ce que** Hy₂ représente l'un des hétérocycles suivants : azétidine, pyrrole, dihydropyrrole, pyrrolidine, furanne, dihydrofuranne, tétrahydrofuranne, thiophène, dihydrothiophène, tétrahydrothiophène, imidazole, dihydroimidazole, imidazolidine, dihydrothiazole, thiazolidine, dihydropyrazole, pyrazolidine, oxazole, dihydrooxazole, oxazolidine, isoxazole, dihydroisoxazole, isoxazolidine, isothiazole, dihydroisothiazole, isothiazolidine, triazole, dihydrotriazole, triazolidine, oxadiazole, dihydrooxadiazole, oxadiazolidine, thiadiazole, dihydrothiadiazole, thiadiazolidine, tétrazole, pyridine, dihydropyridine, tetrahydropyridine, pipéridine, pyranne, dihydropyranne, tétrahydropyranne, pyrimidine, dihydropyrimidine, tétrahydropyrimidine, pipérazine, pyridazine, pyrazine, triazine, morpholine, azépine, diazépine.

12. Utilisation selon l'une des revendications précédentes, **caractérisée en ce que** Hy₃ représente l'un des composés suivants : azétidine, pyrrole, dihydropyrrole, pyrrolidine, furanne, dihydrofuranne, tétrahydrofuranne, thiophène, dihydrothiophène, tétrahydrothiophène, imidazole, dihydroimidazole, imidazolidine, dihydrothiazole, thiazolidine, pyrazole, dihydropyrazole, pyrazolidine, oxazole, dihydrooxazole, oxazolidine, isoxazole, dihydroisoxazole, isoxazolidine, isothiazole, dihydroisothiazole, isothiazolidine, triazole, dihydrotriazole, triazolidine, oxadiazole, dihydrooxadiazole, oxadiazolidine, thiadiazole, dihydrothiadiazole, thiadiazolidine, tétrazole, pyridine, dihydropyridine, tetrahydropyridine, pipéridine, pyranne, dihydropyranne, tétrahydropyranne, pyrimidine, dihydropyrimidine, tétrahydropyrimidine, pipérazine, pyridazine, pyrazine, triazine, morpholine, azépine, diazépine.

13. Utilisation selon l'une des revendications précédentes, **caractérisée en ce que** Hy₅ représente l'un des composés suivants : azétidine, dihydropyrrole, pyrrolidine, dihydrofuranne, tétrahydrofuranne, dihydrothiophène, tétrahydrothiophène, imidazole, dihydroimidazole, imidazolidine, thiazole, dihydrothiazole, thiazolidine, dihydropyrazole, pyrazolidine, oxazole, dihydrooxazole, oxazolidine, isoxazole, dihydroisoxazole, isoxazolidine, isothiazole, dihydroisothiazole, isothiazolidine, triazole, dihydrotriazole, triazolidine, oxadiazole, dihydrooxadiazole, oxadiazolidine, thiadiazole, dihydrothiadiazole, thiadiazolidine, tétrazole, dihydropyridine, tetrahydropyridine, pipéridine, pyranne, dihydropyranne, tétrahydropyranne, pyrimidine, dihydropyrimidine, tétrahydropyrimidine, pipérazine, pyridazine, pyrazine, triazine, morpholine, azépine, diazépine, 2-benzothiazolyle, thiazolo[2,3-c][1,2,4]triazole.

14. Utilisation selon l'une des revendications précédentes, **caractérisée en ce que** Hy₇ représente un cycle azétidine, pyrrole, dihydropyrrole, pyrrolidine, furanne, dihydrofuranne, tétrahydrofuranne, thiophène, dihydrothiophène, tétrahydrothiophène, imidazole, dihydro-imidazole, imidazolidine, thiazole, dihydrothiazole, thiazolidine, pyrazole, dihydropyrazole, pyrazolidine, oxazole, dihydro-oxazole, oxazolidine, isoxazole, dihydro-isoxazole, isoxazolidine, isothiazole, dihydro-isothiazole, isothiazolidine, triazole, dihydrotriazole, triazolidine, oxadiazole, dihydro-oxadiazole, oxadiazolidine, thiadiazole, dihydrothiadiazole, thiadiazolidine, tétrazole, pyridine, dihydropyridine, tétrahydropyridine, pyranne, dihydropyranne, tétrahydropyranne, pyrimidine, dihydropyrimidine, tétrahydropyrimidine, pipérazine, pyridazine, pyrazine, triazine, morpholine, azépine, diazépine, éther couronne à 15 atomes et 5 motifs -CH₂CH₂O-.

15. Utilisation selon l'une des revendications précédentes, **caractérisée en ce que** Hy₁, Hy₆, Hy₈, Hy₉ et Hy₁₀ représentent indépendamment les cycles azétidine, pyrrole, dihydropyrrole, pyrrolidine, furanne, dihydrofuranne, tétrahydrofuranne, thiophène, dihydrothiophène, tétrahydrothiophène, imidazole, dihydro-imidazole, imidazolidine, thiazole, dihydrothiazole, thiazolidine, pyrazole, dihydropyrazole, pyrazolidine, oxazole, dihydro-oxazole, oxazolidine, isoxazole, dihydro-isoxazole, isoxazolidine, isothiazole, dihydro-isothiazole, isothiazolidine, triazole, dihydrotriazole, triazolidine, oxadiazole, dihydro-oxadiazole, oxadiazolidine, thiadiazole, dihydrothiadiazole, thiadiazolidine, tétrazole, pyridine, dihydropyridine, tétrahydropyridine, pipéridine, pyranne, dihydropyranne, tétrahydropyranne, pyrimidine, dihydropyrimidine, tétrahydropyrimidine, pipérazine, pyridazine, pyrazine, triazine, morpholine, azépine, diazépine.

16. Utilisation selon l'une des revendications précédentes, **caractérisée en ce que** le composé présente une des formules (II) et (III) suivantes : 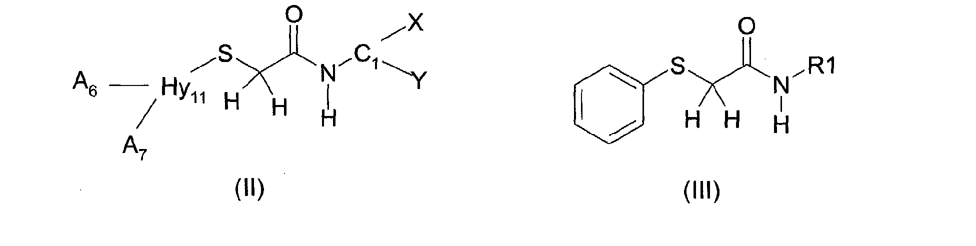 dans lesquelles X et Y représentent indépendamment un atome d'hydrogène, un atome ou groupe halogène ; C₁ représente un cycle hydrocarboné de 3 à 6 à atomes de carbone, saturé ou insaturé ; Hy₁₁ représente un hétérocycle de 5 à 6 atomes contenant au moins un hétéroatome choisi parmi N, S et leur combinaison ; A₆ et A₇ représentent indépendamment un substituant choisi parmi hydrogène, alkyle, COR, OR, SR, CN, COOR et les cycles C₈ à 5 ou 6 atomes, saturés ou non comportant éventuellement de 1 à 4 hétéroatomes choisis parmi S, N et leurs associations et/ou étant éventuellement accolés à un cycle C₉ hydrocarboné de 5 à 6 atomes de carbone ; R₁ représente un radical alkyle en C₁-C₂₀ linéaire ou ramifié, saturé ou insaturé, éventuellement substitué par au moins un substituant A₁, un cycle hydrocarboné ou un hétérocycle Hy₂ éventuellement substitué par au moins un substituant A₃.

17. Utilisation selon l'une des revendications précédentes, **caractérisée en ce que** le sel du composé de formule (I) est un sel choisi parmi les sels de sodium, de potassium, les sels de zinc (Zn²⁺), de calcium (Ca²⁺), de cuivre (Cu²⁺), de fer (Fe²⁺), de strontium (Sr²⁺), de magnésium (Mg²⁺), de manganèse (Mn²⁺), les sels de tri-éthanolamine, mono-éthanolamine, di-éthanolamine, hexadécylamine, N,N,N',N'-tétrakis-(hydroxy-propyl-2) éthylène diamine, tris-hydroxyméthylaminométhane, les hydroxydes et les carbonates.

18. Utilisation selon l'une des revendications précédentes, **caractérisée en ce que** le composé de formule (I) satisfait à l'une des formules suivantes :

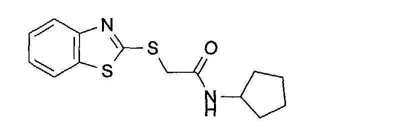

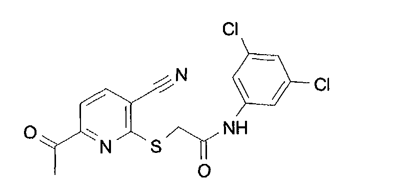

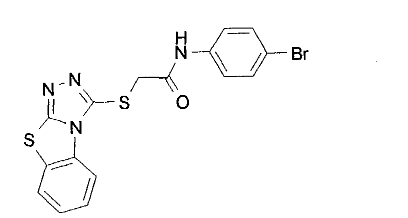

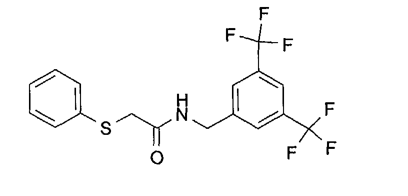

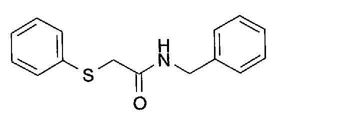

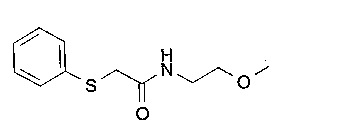

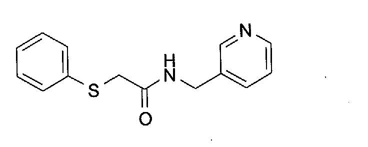

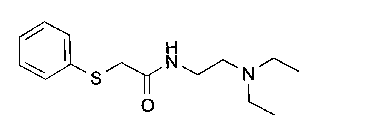

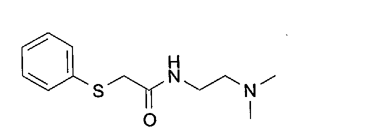

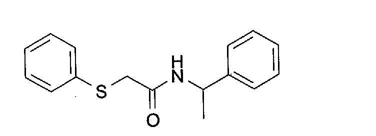

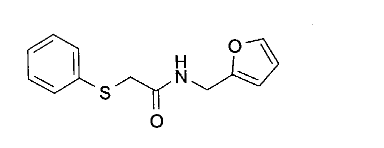

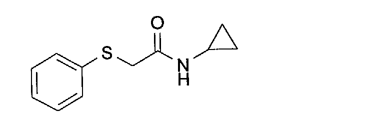

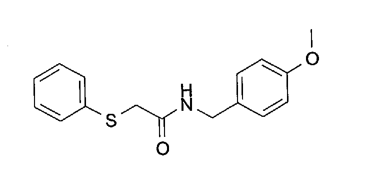

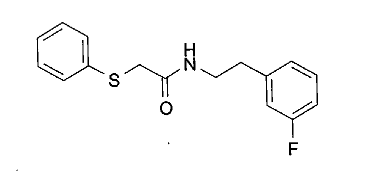

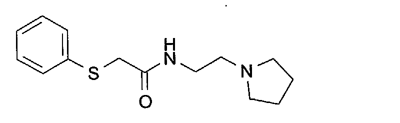

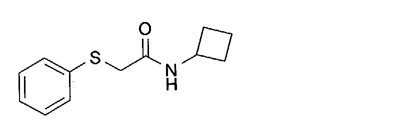

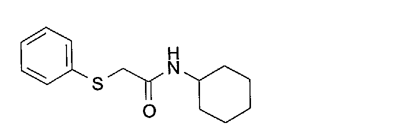

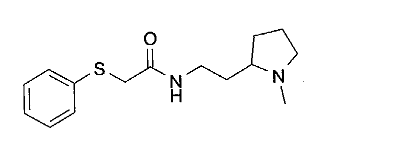

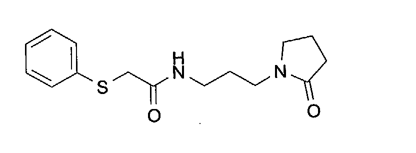

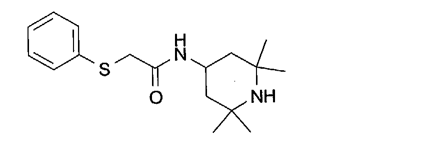

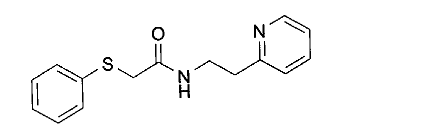

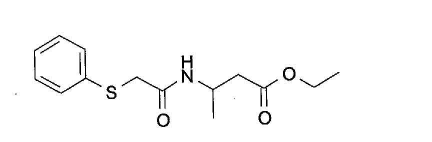

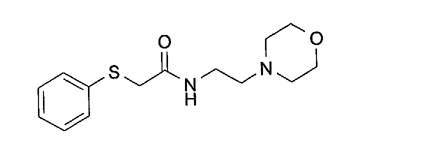

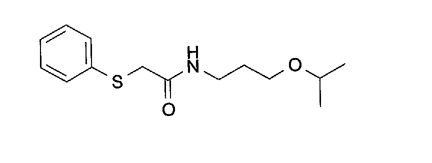

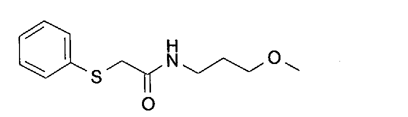

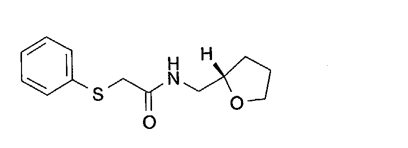

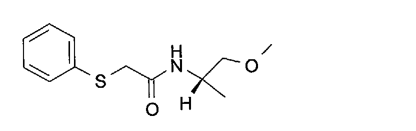

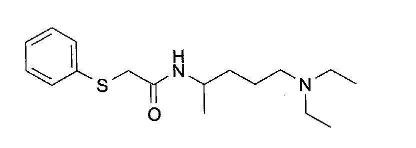

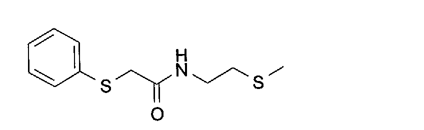

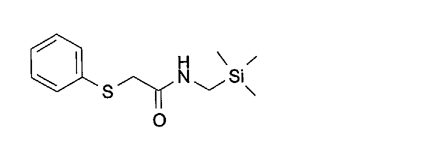

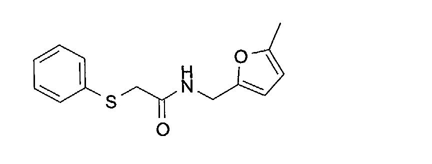

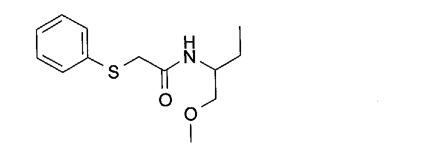

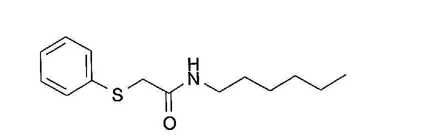

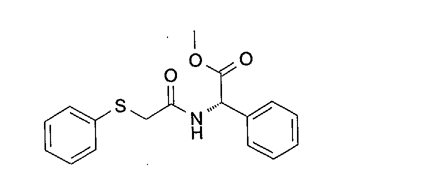

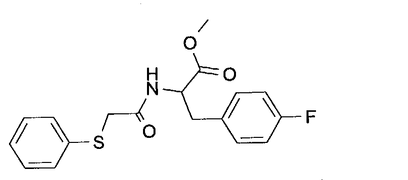

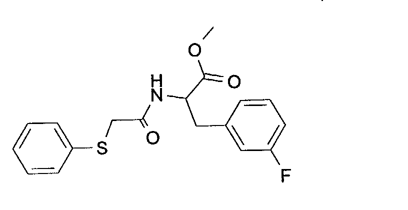

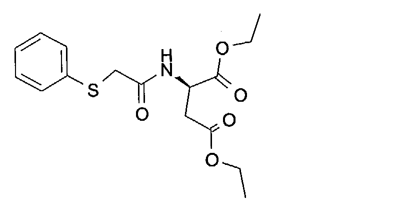

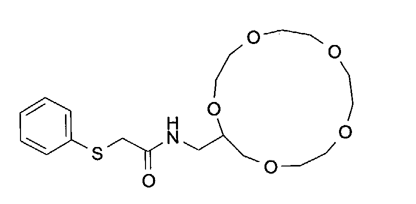

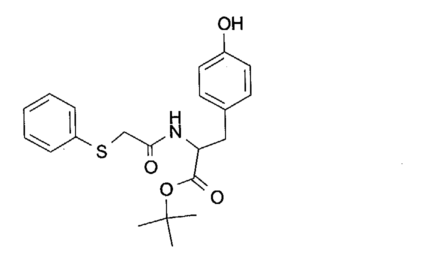

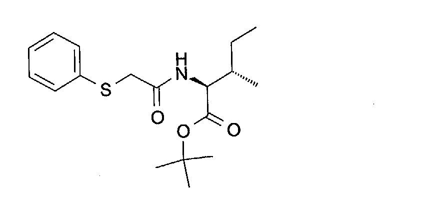

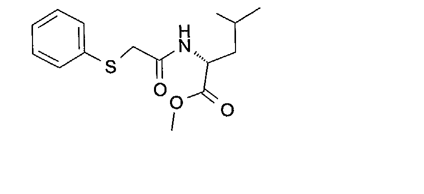

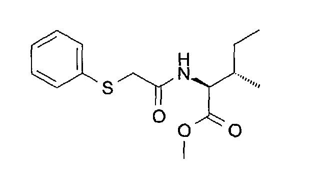

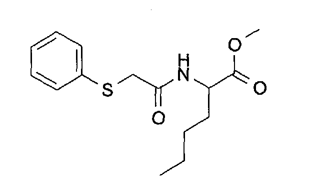

19. Utilisation selon l'une des revendications précédentes, **caractérisée en ce que** le composé de formule (I) ou un mélange de composés de formule (I) est utilisé à une concentration allant de 10⁻³ à 5%, de préférence de 10⁻² à 2%, par rapport au poids total de la composition.

20. Utilisation selon l'une des revendications précédentes, **caractérisée en ce que** la composition est une composition à application topique.

21. Composition de soin ou de maquillage des fibres kératiniques à application topique, contenant un milieu physiologiquement acceptable et une quantité efficace d'au moins un composé 2-thioacétamide de formule (I) ou de l'un de ses sels, 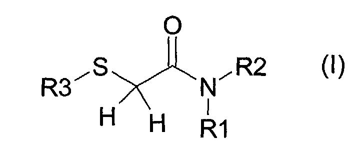 dans laquelle :
**a) R**_{**1**} **et R**_{**2**} représentent indépendamment :
1) un atome d'hydrogène,
2) un radical alkyle en C₁-C₂₀ éventuellement substitué par au moins un substituant A₁,
3) un cycle hydrocarboné C₁, éventuellement accolé à un cycle C₂ contenant éventuellement au moins un hétéroatome pour former un hétérocycle Hy₁, ces cycles C₁ et C₂ pouvant comporter au moins une fonction carbonyle ou thiocarbonyle et être substitués par au moins un substituant A₃,
4) un hétérocycle Hy₂ éventuellement accolé à un cycle C₂ contenant éventuellement au moins un hétéroatome pour former un hétérocycle Hy₃, ces cycles Hy₂ et C₂ pouvant comporter une fonction carbonyle ou thiocarbonyle et être substitués par au moins un substituant A₃, ou
5) un groupe C(=NR)R', C(=NR)NR'R", COR, CSR, COOR, CONRR', SO₂R ou SO₂NRR' ;
**b) R**_{**3**} représente :
1) un radical alkyle en C₁-C₂₀ éventuellement substitué par au moins un substituant A₂,
2) un cycle hydrocarboné C₃, éventuellement accolé à un cycle C₄ contenant éventuellement au moins un hétéroatome pour former un hétérocycle Hy₃, ces cycles C₃ et C₄ pouvant comporter au moins une fonction carbonyle ou thiocarbonyle et être substitués par au moins un substituant A₃,
3) un hétérocycle Hy₄ représentant un cycle pyrrole, furanne, thiophène ou pyrazole, éventuellement accolé à un cycle C₅ représentant un cycle phényle, pyridine ou pyrimidine, ces deux cycles Hy₄ et C₅ pouvant être substitués par au moins un substituant A₃,
4) un cycle pyridine éventuellement accolé à un cycle C₂ contenant éventuellement au moins un hétéroatome pour former un hétérocycle Hy₁, ces cycles pyridine et C₂ pouvant comporter au moins une fonction carbonyle ou thiocarbonyle et être substitués par au moins un substituant A₄,
5) un hétérocycle Hy₅ différent de Hy₄ et du cycle pyridine, éventuellement accolé à un cycle C₂ contenant éventuellement au moins un hétéroatome pour former un hétérocycle Hy₁, ces cycles Hy₅ et C₂ pouvant comporter au moins une fonction carbonyle ou thiocarbonyle et être substitués par au moins un substituant A₃, ou
6) un groupe C(=NR)R', C(=NR)NR'R", COR, CSR, COOR ou CONRR' ;
**c) A**_{**1**} représente :
1) un halogène,
2) un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R",
3) un cycle hydrocarboné C₆, éventuellement accolé à un cycle C₇ contenant éventuellement au moins un hétéroatome pour former un hétérocycle Hy₆, ces cycles C₆ et C₇ pouvant comporter au moins une fonction carbonyle ou thiocarbonyle et être substitués par au moins un substituant A₅,
4) un hétérocycle Hy₇ éventuellement accolé à un cycle C₇ contenant éventuellement au moins un hétéroatome pour former un hétérocycle Hy₆, ces cycles Hy₇ et C₇ pouvant comporter au moins une fonction carbonyle ou thiocarbonyle et être substitués par au moins un substituant A₅ ;
**d) A**_{**2**} représente :
1) un halogène,
2) un groupe CF₃, CN, OR, SR, NRR', NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R", ou
3) un cycle C₈ éventuellement accolé à un cycle C₉, ces cycles C₈ et C₉ contenant éventuellement au moins un hétéroatome pour former un hétérocycle Hy₈ et/ou au moins une fonction carbonyle ou thiocarbonyle et/ou étant substitués par au moins un substituant A₅ ;
**e) A**_{**3**} représente :
1) A₂ , ou
2) un groupe alkyle en C₁-C₂₀ éventuellement substitué par au moins un substituant A₅ ;
**f) A**_{**4**} représente :
1) un halogène,
2) un groupe CF₃, CN, OR, SR, NRC(=NR')NR"R"', COR, CSR, COOR, CONRR', CSNRR', NRCSR', NRCSNR'R", NRCOR', NRCONR'R", SO₂NRR', NRSO₂R', SO₂R ou SiRR'R",
3) un groupe alkyle en C₁-C₂₀ éventuellement substitué par au moins un substituant A₅,
4) un cycle C₈ éventuellement accolé à un cycle C₉, ces cycles C₈ et C₉ contenant éventuellement au moins un hétéroatome pour former un hétérocycle Hy₈ et/ou au moins une fonction carbonyle ou thiocarbonyle et/ou étant substitués par au moins un substituant A₅ ;
**g) A**_{**5**} représente :
1) un halogène,
2) un groupe CF₃, CN, OR₅, SR₅, NR₅R₅', NR₅C(=NR₅')NR₅"R₅"', COR₅, CSR₅, COOR₅, CONR₅R₅', CSNR₅R₅', NR₅CSR₅', NR₅CSNR₅'R₅", NR₅COR₅', NR₅CONR₅'R₅", SO₂NR₅R₅', NR₅SO₂R₅', SO₂R₅ ou SiR₅R₅'R₅", R₅, R₅', R₅" étant un atome d'hydrogène ou un groupe alkyle en C₁-C₂₀,
3) un groupe alkyle en C₁-C₂₀, ou
4) un cycle C₁₀ éventuellement accolé à un autre cycle C₁₁, ces cycles contenant éventuellement au moins un hétéroatome pour former un hétérocycle Hy₉ et/ou comportant au moins une fonction carbonyle ou thiocarbonyle ;
**h) R, R', R" et R"'**, identiques ou différents, représentent :
1) un atome d'hydrogène,
2) un groupe alkyle en C₁-C₂₀ éventuellement substitué par au moins un substituant A₅, ou
3) un cycle C₁₂ éventuellement accolé à un autre cycle C₁₃, ces cycles contenant éventuellement au moins un hétéroatome pour former un hétérocycle Hy₁₀ et pouvant être substitués par au moins un substituant A₅ ;
**i) Hy**_{**1**} **à Hy**_{**10**} représentent indépendamment un hétérocycle pouvant contenir de 1 à 4 hétéroatomes choisis parmi N, O et S.

22. Composition selon la revendication 21, **caractérisée en ce que** le composé présente la formule (II) ou (III) suivante : 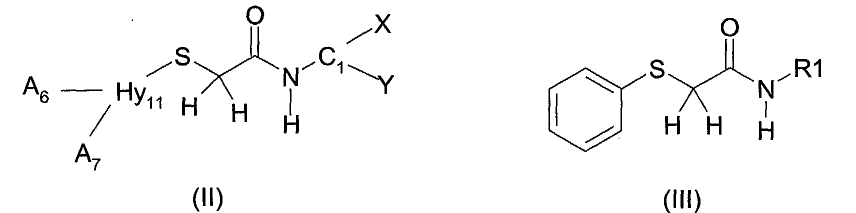 dans lesquelles X et Y représentent indépendamment un atome d'hydrogène, un atome ou groupe halogène ; C₁ représente un cycle hydrocarboné de 3 à 6 à atomes de carbone, saturé ou insaturé ; Hy₁₁ représente un hétérocycle de 5 à 6 atomes contenant au moins un hétéroatome choisi parmi N, S et leur combinaison ; A₆ et A₇ représentent indépendamment un substituant choisi parmi hydrogène, alkyle, COR, OR, SR, CN, COOR et les cycles C₈ à 5 ou 6 atomes, saturés ou non comportant éventuellement de 1 à 4 hétéroatomes choisis parmi S, N et leurs associations et/ou étant éventuellement accolés à un cycle C₉ hydrocarboné de 5 à 6 atomes de carbone ; R₁ représente un radical alkyle en C₁-C₂₀ linéaire ou ramifié, saturé ou insaturé, éventuellement substitué par au moins un substituant A₁, un cycle hydrocarboné ou un hétérocycle Hy₂ éventuellement substitué par au moins un substituant A₃.

23. Composition selon l'une des revendications 21 à 22, **caractérisée en ce que** l'un au moins des R₁ et R₂ représentent un atome d'hydrogène ; un radical alkyle en C₁-C₂₀ dont au moins un hydrogène est substitué par au moins un substituant A₁ ; un cycle hydrocarboné saturé ou non de 3 à 6 atomes de carbone, dont au moins un hydrogène est éventuellement substitué par des atomes d'halogène ou CF₃.

24. Composition selon l'une des revendications 21 à 23, **caractérisée en ce que** R₁ représente H et R₂ représente un radical cyclopropyle, cyclobutyle, cyclopentyle, cyclohexyle ou un radical phényle substitué par un atome de brome ou 2 atomes de chlore.

25. Composition selon l'une des revendications 21 à 23, **caractérisée en ce que** R₁ représente H et R₂ représente un hétérocycle de 5 à 6 atomes, saturé ou insaturé, contenant de 1 à 4 hétéroatomes choisis parmi N, O, S et éventuellement substitué par au moins un radical alkyle, CF₃, OR, SR, NRR', COR, COOR, CONRR' ou NRCOR' avec R et R' représentant H ou alkyle.

26. Composition selon la revendication 25, **caractérisée en ce que** R₂ représente un cycle pipéridine éventuellement substitué par au moins un radical alkyle en C₁-C₁₀.

27. Composition selon l'une des revendications 21 à 23 **caractérisée en ce que** R₁ représente H et R₂ représente un alkyle en C₁-C₂₀ saturé, éventuellement substitué par au moins un substituant A₁.

28. Composition selon la revendication 27, **caractérisée en ce que** A₁ représente un cycle de 5 à 6 atomes, saturé ou insaturé, contenant éventuellement de 1 à 4 hétéroatomes choisis parmi N, O, S, pouvant contenir au moins une fonction carbonyle et être éventuellement substitué par au moins un groupe choisi parmi alkyle ; F, CF₃, OR, SR, NRR', COR, COOR, CONRR' ou NRCOR' avec R et R' représentant H ou alkyle, un cycle éther-couronne à 15 atomes et 5 motifs -CH₂CH₂O- ; un groupe choisi parmi CF₃, OR, SR, NRR', COR, COOR, CONRR', NRCOR' ou SiRR'R" avec R, R' et R" représentant H ou alkyle.

29. Composition selon l'une des revendications 21 à 28, **caractérisée en ce que** R₃ représente un phényle ou un hétérocycle de 5 à 6 atomes, substitué par au moins un substituant A₃ ou accolé à un cycle hydrocarboné ou hétérocyclique de 5 à 6 atomes.

30. Composition selon l'une des revendications 21 à 29, **caractérisée en ce que** R₃ représente un groupe phényle, 2-benzothiazole, 2-pyridyle, 6-acétyl-nicotinonitrile, triazole ou 4a,8a-dihydro-benzo[4,5]thiazolo[2,3-c][1,2,4]triazolyle.

31. Composition selon l'une des revendications 21 à 30, **caractérisée en ce que** le sel du composé de formule (I) est un sel choisi parmi les sels de sodium, de potassium, les sels de zinc (Zn²⁺), de calcium (Ca²⁺), de cuivre (Cu²⁺), de fer (Fe²⁺), de strontium (Sr²⁺), de magnésium (Mg²⁺), de manganèse (Mn²⁺), les sels tri-éthanolamine, mono-di-éthanolamine, éthanolamine, hexadécylamine, N,N,N',N'-tétrakis-(hydroxy-propyl-2) éthylène diamine, tris-hydroxyméthylaminométhane, les hydroxydes et les carbonates.

32. Composition selon l'une des revendications 21 à 31, **caractérisée en ce que** le composé de formule (I) satisfait à la formule suivante :

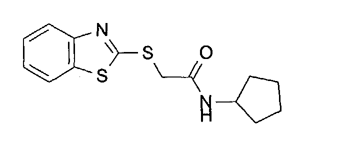

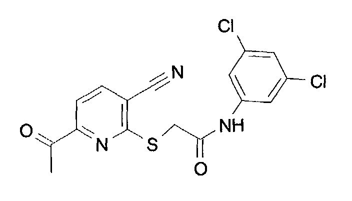

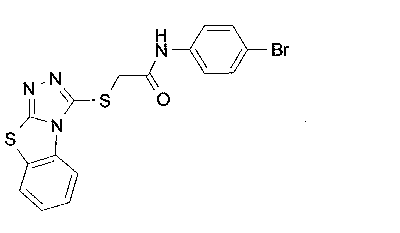

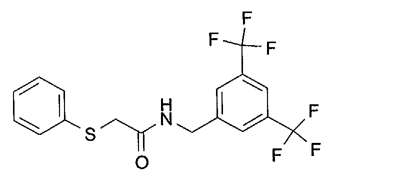

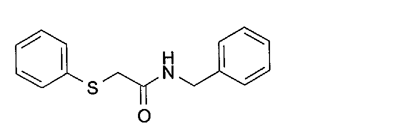

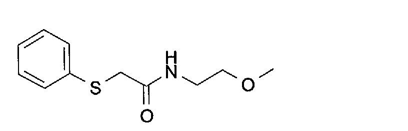

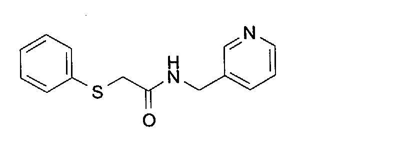

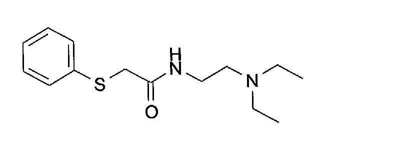

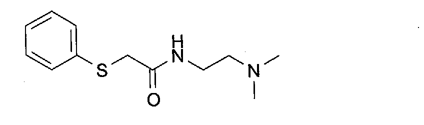

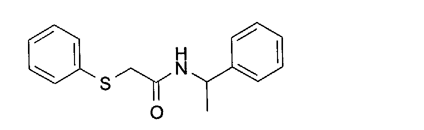

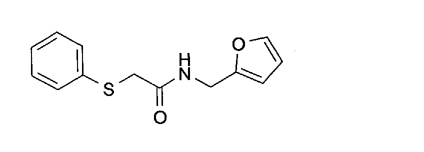

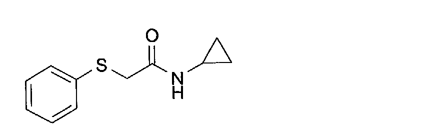

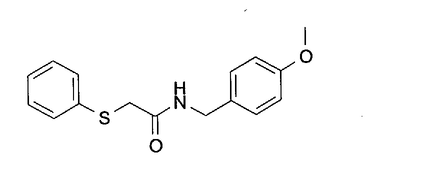

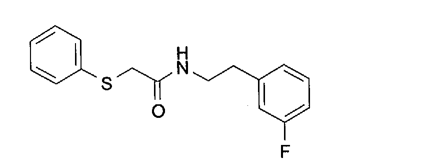

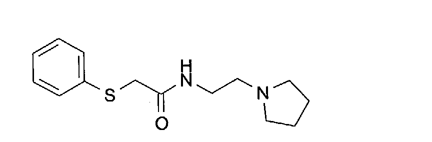

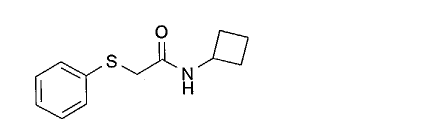

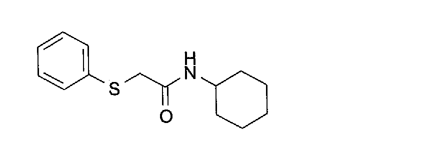

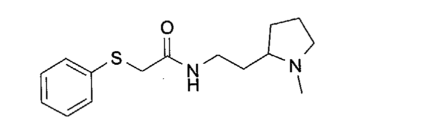

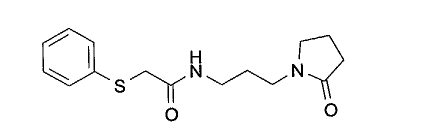

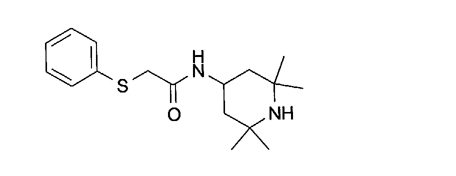

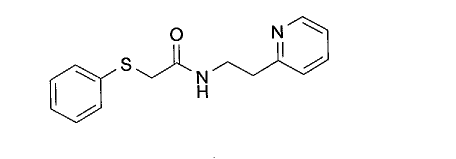

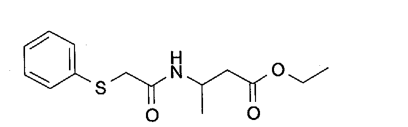

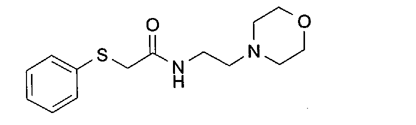

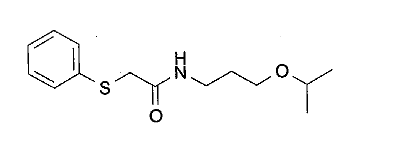

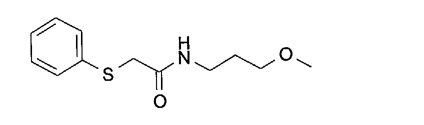

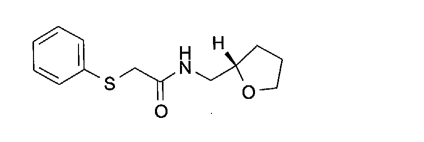

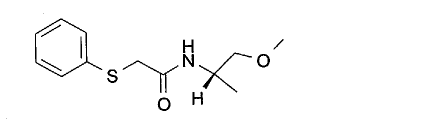

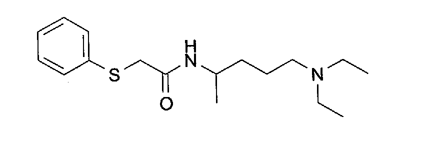

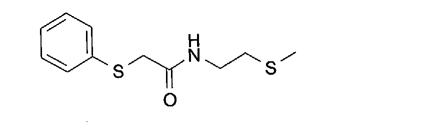

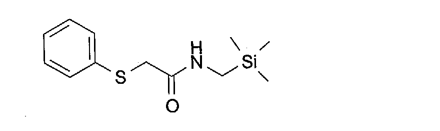

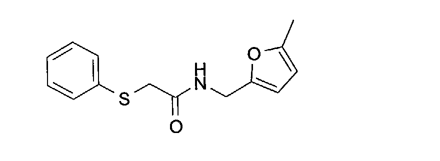

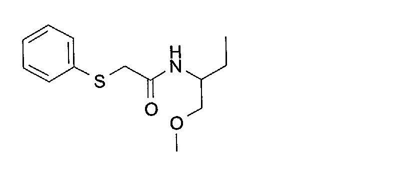

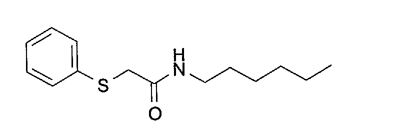

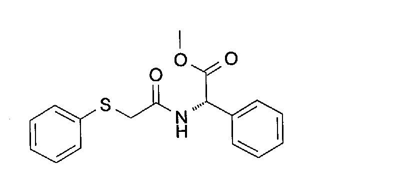

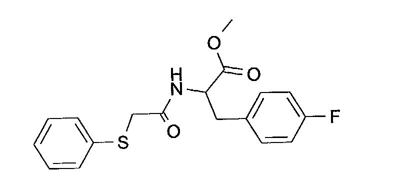

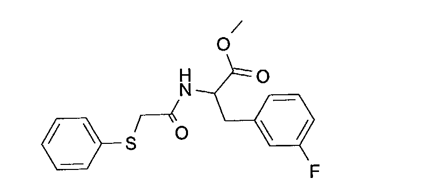

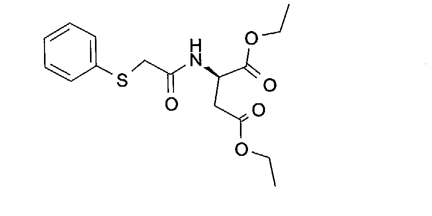

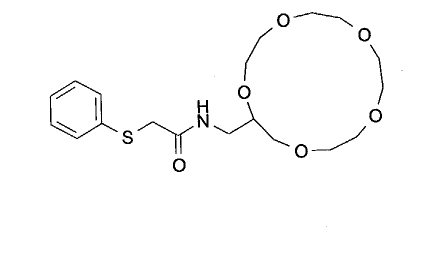

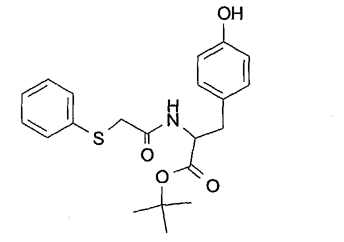

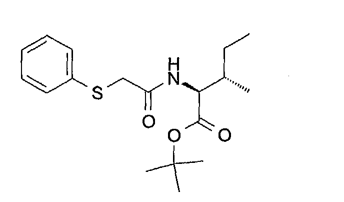

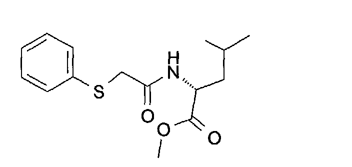

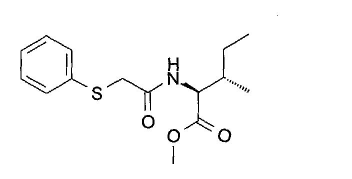

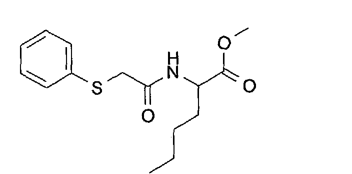

33. Composition selon l'une des revendications 21 à 32, **caractérisée en ce que** le composé de formule (I) ou un mélange de composés de formule (I) est utilisé à une concentration allant de 10⁻³ à 5%, de préférence de 10⁻² à 2%, par rapport au poids total de la composition.

34. Composition selon l'une des revendications 21 à 33, **caractérisée en ce qu'**elle se présente sous forme de crème ou lotion capillaire, de shampooing, d'après-shampooing, de mascara capillaire ou pour cils.

35. Composition selon l'une des revendications 21 à 34, **caractérisée en ce qu'**elle est sous forme de solution ou suspension aqueuse, alcoolique ou hydro-alcoolique.

36. Composition selon l'une des revendications 21 à 35, **caractérisée en ce qu'**elle contient d'autres ingrédients choisis parmi les solvants, les épaississants ou gélifiants de phase aqueuse ou de phase huileuse, les matières colorantes solubles dans le milieu de la composition, les charges, les pigments, les antioxydants, les conservateurs, les parfums, les électrolytes, les neutralisants, les polymères filmogènes, les agents bloqueurs d'U.V., les actifs cosmétiques et pharmaceutiques autres que les composés de formule (I), leurs mélanges.

37. Composition selon l'une des revendications 21 à 36, **caractérisée en ce qu'**elle contient au moins un composé additionnel actif favorisant la repousse et/ou limitant la chute des fibres kératiniques.

38. Composition selon la revendication 37, **caractérisée en ce que** l'actif additionnel est choisi parmi l'aminexil, le 6-0-[(9Z,12Z)-octadéca-9,12-diènoyl]hexapyranose, les inhibiteurs de lipoxygénase, les inhibiteurs de bradykinine, les prostaglandines et leurs dérivés, les agonistes ou antagonistes des récepteurs des prostaglandines, les analogues non prostanoïques de prostaglandines, les vasodilatateurs, les antiandrogènes, les cyclosporines et leurs analogues, les antimicrobiens, les anti-inflammatoires, les rétinoïdes, le chlorure de benzalkonium, le chlorure de benzéthonium, le phénol, l'oestradiol, le maléate de chlorphéniramine, les dérivés de chlorophylline, le cholestérol, la cystéine, la méthionine, le menthol, l'huile de menthe poivrée, le panthoténate de calcium, le panthénol, le résorcinol, les activateurs de la protéine kinase C, les inhibiteurs de la glycosidase, les inhibiteurs de glycosaminoglycanase, les esters d'acide pyroglutamique, les acides hexosaccharidiques ou acyl-hexosaccharique, les éthylènes aryle substitués, les amino-acides N-acylés, les flavonoïdes, les dérivés et analogues d'ascomycine, les antagonistes d'histamine, les saponines, les inhibiteurs de protéoglycanase, les agonistes et antagonistes d'estrogènes, les pseudotérines, les cytokines et les promoteurs de facteurs de croissance, les inhibiteurs d'IL-1 ou d'IL-6, les promoteurs d'IL-10, les inhibiteurs de TNF, les vitamines, les benzophénones, l'hydantoïne, l'acide rétinoïque, les agents antiprurigineux, les antiparasitaires, les antifongiques, les agents antagonistes de calcium, les hormones, les triterpènes, les agents antiandrogènes, les inhibiteurs stéroïdiens ou non stéroïdiens des 5-α-réductases, les agonistes de canaux potassiques, les antagonistes du récepteur FP, leurs mélanges.

39. Composition selon l'une des revendications 21 à 38, **caractérisée en ce qu'**elle contient, en outre, au moins un actif choisi parmi les protéines, les hydrolysats de protéine, les acides aminés, les polyols, l'urée, l'allantoïne, les sucres et dérivés de sucre, les vitamines hydrosolubles, les extraits végétaux, les hydroxy-acides, le rétinol, le tocophérol, les dérivés du rétinol ou du tocophérol, les acides gras essentiels, les céramides, les huiles essentielles, les dérivés de l'acide salicylique comme le n-octanoyl-5 salicylique, les esters des hydroxy-acides, les phospholipides, leurs mélanges.

40. Procédé de traitement cosmétique des fibres kératiniques humaines et/ou de la peau d'où émergent lesdites fibres, **caractérisé par le fait qu'**il consiste à appliquer sur lesdites fibres et/ou ladite peau, une composition cosmétique telle que définie dans l'une quelconque des revendications 21 à 39, à laisser celle-ci en contact avec lesdites fibres kératiniques et/ou ladite peau, et éventuellement à rincer.

41. Procédé de soin cosmétique et/ou de maquillage des cils humains, en vue d'améliorer leur état et/ou leur aspect, **caractérisé en ce qu'**il consiste à appliquer sur les cils et/ou les paupières une composition de mascara comprenant au moins un composé de formule (I) ou l'un de ses sels et à laisser celle-ci au contact des cils et/ou des paupières.

42. Procédé de soin cosmétique des cheveux et/ou du cuir chevelu humains, en vue d'améliorer leur état et/ou leur aspect, **caractérisé en ce qu'**il consiste à appliquer sur les cheveux et/ou le cuir chevelu, une composition cosmétique comprenant une quantité efficace d'au moins un composé de formule (I) ou l'un de ses sels, à laisser celle-ci en contact avec les cheveux et/ou le cuir chevelu, et éventuellement à rincer les cheveux et/ou le cuir chevelu.

43. Composition de soin ou de maquillage des fibres kératiniques, comprenant dans un milieu physiologiquement acceptable, en particulier cosmétique, au moins un composé de formule (I) ou l'un de ses sels et au moins un actif additionnel favorisant la repousse des fibres kératiniques humaines et/ou limitant leur chute choisi parmi l'aminexil, les agonistes du récepteur FP et les vasodilatateurs.

44. Composition selon la revendication 43, **caractérisée en ce que** l'actif additionnel est choisi parmi l'aminexil, le minoxidil, le latanoprost et le travoprost.
